# EUROPÄISCHE PATENTSCHRIFT

(11) **EP 0 136 259 B1**
(45) Veröffentlichungstag und Bekanntmachung des Hinweises auf die Patenterteilung: **15.06.1988**
(21) Anmeldenummer: 84810374.3
(22) Anmeldetag: 30.07.1984
(51) Int. Cl.: C07D 271/06, C07D 271/10, C07D 271/08, C07D 251/22, C07D 239/26, C07D 239/30, C07D 239/32, C07D 239/34, C07D 239/38, C07D 239/42, C07D 239/69

(54) **4-Heterocyclylvinyl-4'-styryl-biphenyle**
4-Heterocyclyl-vinyl-4'-styryl-biphenyls
Hétérocyclyl-4-styryl-4' biphényles

(30) Priorität: 05.08.1983 CH 4266/83
(43) Veröffentlichungstag der Anmeldung: 03.04.1985
(73) Patentinhaber: CIBA-GEIGY AG, 4002 Basel (CH)
(72) Erfinder: Meyer, Hans Rudolf, Dr., CH-4102 Binningen (CH)

(56) Entgegenhaltungen:
- EP-A- 0 031 796
- CH-A- 619 336
- DD-A- 153 714
- DE-A- 2 712 686
- GB-A- 1 360 142
- CHEMICAL ABSTRACTS, Vol. 98, No. 10, 7. März 1983, Columbus, Ohio, USA. GALUNOV, N.Z.; GUNDER, O.A.; KOPINA, I.V.; TSIRLIN, YU.A.: "Study of the shape of scintillation light pulses of fast-response plastic scintillators based on polyvinylxlene". Seite 480, Spalte 2, Zusammenfassung Nr. 80 124e
- CHEMICAL ABSTRACTS, Vol. 88, No. 8, 20. Februar 1978, Columbus, Ohio, USA. SIEGRIST, ADOLF EMIL; KORMANY, GEZA; KEBAS, GUGLIELMO; SCHLAEPFER HANS: "Anil synthesis. Part 15. Preparation of heterocyclic substituted stilbenyl derivatives of 2H-1,2,3-triazole". Seite 56, Spalte 2, Zusammenfassung Nr. 51 934g
- CHEMICAL ABSTRACTS, Vol. 97, No. 11, 13. September 1982, Columbus, Ohio, USA. KRASOVITSKII, B.M.; EGOROVA, N.P.; AFANASIADI, L. SH.; LYSOVA, I.V.: POLYAKOV, V.K.; TSUKERMAN, S.V.: "Synthesis and spectral-luminescent properties of hetarylethylene derivatives of 2,5-diphenyl-oxazole and 2,5-diphenyl-1,3,4-oxadiazole". Seite 765, Spalte 1, Zusammenfassung Vr. 92 212z
- CHEMICAL ABSTRACTS, Vol. 92, No. 6, 11. Februar 1980, Columbus, Ohio, USA. PALMBERG, ROGER B.; SIEGRIST, ADOLF EMIL: "Anil sythesis. Part 21. Preparation of stilbenyl derivatives of pyrazole". Seite 64, Spalte 2, Zusammenfassung Nr. 43 264f

## Beschreibung

Die vorliegende Erfindung betrifft neue 4-Heterocyclylvinyl-4'-styryl-biphenyle, Verfahren zu deren Herstellung und deren Verwendung zum optischen Aufhellen von synthetischen, halbsynthetischen und natürlichen-hochmolekularen organischen Materialien sowie Mittel, die diese Verbindungen enthalten.

Symmetrische 4,4'-Bis-(oxadiazolylvinyl) biphenyle, die als optische Aufheller u. a. für Polyester verwendet werden können, sind aus der DE-A-2 350 570 bekannt. In der Praxis eignen sich diese Aufheller hauptsächlich für Spinnmassen, auf Textilien erreichen sie nur ungenügende Weisseffekte.

Symmetrische 4,4'-Bis(triazolylvinyl)-biphenyle sind ebenfalls als optische Aufheller bekannt. Siehe dazu DE-A-2 262 578, DE-A-2 212 480, DE-A-2 262 633. Die beiden erstgenannten DE-A umfassen auch unsymmetrische 4-Triazolylvinyl-4'-styryl-biphenyle, jedoch konkret sind solche Verbindungen nicht beschrieben. Die in der DE-A-2 262 633 beschriebenen 4-Triazolylvinyl-4'-styryl-biphenyle sind mit mindestens einer Sulfogruppe substituiert. Sie sind nur auf Baumwollsubstraten als optische Aufheller brauchbar.

Ferner sind noch die aus der DE-A-2 647179 bekannten 4-0xadiazolyl-vinyl-4'-styrylbenzole zu erwähnen, die ebenfalls als optische Aufheller, insbesondere für Polyester, brauchbar sind. Sie erzielen aber keine allzu hohen Weisseffekte und ihre Lichtechtheit genügt nicht vollständig den praktischen Anforderungen.

Aufgabe der vorliegenden Erfindung war es, neue, als optische Aufheller brauchbare Verbindungen zu schaffen, die insbesondere auf Polyester und auch auf Polyamid besonders günstige Aufhelleffekte liefern, die gute Aufzieheigenschaften und Lichtechtheiten aufweisen und die verschiedene Nachteile der oben genannten bekannten Verbindungen des Standes der Technik nicht aufweisen.

Es wurde überraschenderweise gefunden, dass bestimmte 4-Heterocyclylvinyl-4'-styryl-biphenyle diesen Anforderungen genügen und somit die Aufgabe der Erfindung lösen. Sie sind als optische Aufheller auch besonders ausgiebig.

Die erfindungsgemässen 4-Heterocyclylvinyl-4'-styryl-biphenyle entsprechen der Formel 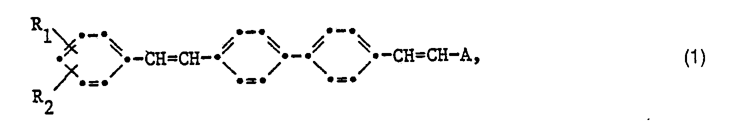 worin A einen unsubstituierten oder nicht-chromophor substituierten Isoxazolyl-, Oxadiazolyl-, Pyrimidinyl- oder Triazinylrest, R₁ Wasserstoff oder einen nicht-chromophoren Substituenten und R₂ Wasserstoff, Halogen oder Alkyl bedeuten.

Als nicht-chromophore Substituenten in Verbindungen der Formel (1) kommen vor allem solche in Betracht, die auf dem Gebiet der optischen Aufheller üblich sind. Beispiele hierfür sind : unsubstituiertes oder nicht-chromophor substituiertes Alkyl oder Alkoxy, Alkenyl, Cycloalkyl, Aryl, Aralkyl, Pyridyl, gegebenenfalls substituiertes Aminocarbonyl und Alkoxycarbonyl, Cyano, Alkylsulfonyl, Alkoxysulfonyl, gegebenenfalls substituiertes Aminosulfonyl, Acyl, Acylamino, Hydroxy, Alkylmercapto, Aryloxy, Aralkoxy, Alkenyloxy, Aryloxycarbonyl, Aryloxysulfonyl, Aralkoxycarbonyl, Carboxy, Sulfo, Halogen, Acyloxy, Trifluormethyl, Amino, Mono- oder Dialkylamino, Alkoxyalkyl.

Unter « Aryl ist vorzugsweise gegebenenfalls nicht-chromophor substituiertes Phenyl wie z. B. Phenyl, Tolyl oder Chlorphenyl zu verstehen. In zusammengesetzen Gruppen (z. B. Aryloxy, Aralkyl, Aralkoxy usw.) gilt für Aryl dieselbe bevorzugte Definition.

Nicht-chromophore Substituenten für Alkylgruppen oder Alkoxygruppen sind beispielsweise : Hydroxy, Alkoxy, Alkoxyalkoxy, Hydroxyalkoxy, Halogen, Cyano, Aryl (insbesondere Phenyl), Sulfo, Carboxy, Carbalkoxy, Aminocarbonyl.

Falls Arylgruppen (oder Arylgruppen in zusammengesetzten Resten), insbesondere Phenylreste (oder Phenylreste in zusammengesetzten Resten wie Phenoxy, Phenylalkyl, Phenylsulfonyl usw.) substituiert sind, tragen sie vorzugsweise einen oder zwei Substituenten aus der Gruppe Halogen (insbesondere Chlor), Alkyl und/oder Alkoxy, ferner Sulfo oder Carboxy und deren Derivate, Cyano und Acyl. Bevorzugte Substituenten sind Chlor, Methyl und Methoxy, von denen bis zu 3 im Ring vorhanden sein können.

Halogen ist insbesondere Fluor, Chlor oder Brom, vorzugsweise Chlor.

Acyl ist insbesondere Alkylcarbonyl, Alkylsulfonyl, und gegebenenfalls durch Alkyl, Alkoxy oder Halogen substituiertes Phenylsulfonyl oder Benzoyl.

Alkyl- und Alkoxygruppen haben als solche oder in Alkyl- oder Alkoxygruppen enthaltenden zusammengesetzen Gruppen in der Regel 1 bis 8, insbesondere 1 bis 6, vorzugsweise 1 bis 4 C-Atome. Cycloalkyl weist im Ring vorzugsweise 5 oder 6 C-Atome auf: Alkenylgruppen haben vorzugsweise 2 bis 6, insbesondere 3 oder 4 C-Atome. Alkylreste in Carbonsäureester- oder-amidgruppen oder in Sulfonamidgruppen haben vorzugsweise 1 bis 4 C-Atome.

Unter « Sulfo und « Carboxy sind die Gruppen -S0₃H und -COOH sowie deren Salze zu verstehen. Bevorzugte Salze dieser Gruppen sind die Alkalimetallsalze und Ammoniumsalze, wobei unter Ammoniumsalzen auch jene Salze zu verstehen sind, die sich von organischen Aminen (« Aminsalze », « substituierte Ammoniumsalze ») ableiten. Besonders bevorzugte Salze sind die Natrium- und Kaliumsalze.

Bevorzugte substituierte Aminocarbonyl-, Alkoxycarbonyl- bzw. Aminosulfonylgruppen entsprechen den Formeln ―CONY₁Y₂, ―COOY₁ bzw. ―SO₂NY₁Y₂, worin Y₁ und Y₂ unabhängig voneinander für Wasserstoff, Alkenyl, Cycloalkyl, unsubstituiertes oder nicht-chromophor substituiertes Alkyl, Phenyl, Phenylalkyl, oder Y₁ und Y₂ zusammen mit dem Stickstoffatom, an das sie gebunden sind, für einen 5-oder 6-gliedrigen gesättigten heterocyclischen Ring stehen, der gegebenenfalls noch zusätzlich 1 oder 2 Heteroatome als Ringglieder enthalten kann und der gegebenenfalls durch Alkylgruppen substituiert sein kann.

Falls 5- oder 6-gliedrige gesättigte heterocyclische Ringe (Y₁ +Y₂) noch weitere Heteroatome im Ring enthalten, können dies insbesondere 1 oder 2 Stickstoff- oder/und Sauerstoffatome sein. Als bevorzugte Heterocyclen, die Y₁ und Y₂ gemeinsam mit dem Stickstoffatom bilden können, sind der Piperidin-, Piperazin-, Morpholin-, Pyrrolidin-, Imidazolidin- und Oxazolidinring zu nennen.

In bevorzugten 4-Heterocyclylvinyl-4'-styryl-biphenylen der Formel (1) bedeutet R₁ Wasserstoff oder einen nicht-chromophoren Substituenten 2. Ordnung. In besonders vorteilhaften Verbindungen der Formel (1) stellt R₁ einen nicht-chromophoren Substituenten 2. Ordnung dar.

Nicht-chromophore Substituenten 2. Ordnung sind die in der organischen Chemie bekannten elektronenanziehenden Substituenten, beispielsweise Acylreste organischer Carbon- oder Sulfonsäuren, Cyano, Trifluormethyl, die Carboxy- und Sulfogruppe und deren funktionelle Derivate wie z. B. deren Salze, Ester und Amide, sowie Derivate von Resten von Phosphor-Sauerstoff-Verbindungen.

Von den Acylresten sind insbesondere zu nennen : Alkylcarbonyl, Alkylsulfonyl, gegebenenfalls durch Alkyl, Alkoxy oder/und Halogen substituiertes Phenylsulfonyl oder Benzoyl. Bevorzugte Salze von Carboxy- und Sulfogruppen wurden bereits weiter oben aufgezählt. Ester der Sulfogruppe könne beispielsweise Alkoxysulfonyl und gegebenenfalls mit Alkyl, Alkoxy und/oder Halogen substituiertes Phenoxysulfonyl sein. Bevorzugte Ester von Carboxylgruppen bzw. Amide von Carboxyl- und Sulfogruppen entsprechen den oben definierten Formeln COOY₁ bzw. CONY₁Y₂ und SO₂NY₁Y₂. Als Beispiel für Derivate von Resten von Phosphor-Sauerstoffverbindungen sei eine Gruppe der Formel 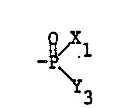 erwähnt, worin X₁ und Y₃ unabhängig voneinander für Halogen, Alkyl, Alkenyl, Phenyl, Phenylalkyl, Hydroxy, Alkoxy, Phenylalkoxy, Cycloalkoxy, Phenoxy, Amino, Mono- oder Dialkylamino, Phenylalkylamino, Acylamino, Phenylamino, Cycloalkylamino, Morpholino, Piperidino oder Pyrrolidino stehen.

In den vorstehend definierten Formeln haben die C-Ketten die weiter oben angegebenen bevorzugten Kettenlängen. Nicht-chromophore Substituenten von Alkylgruppen sind ebenso weiter oben aufgezählt wie bevorzugte gesättigte Heterocyclen Y₁ + Y₂.

Der Substituent A stellt in den Verbindungen der Formel (1) vorzugsweise folgende heterocyclischen Gruppen dar:

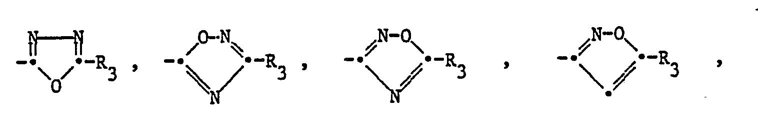

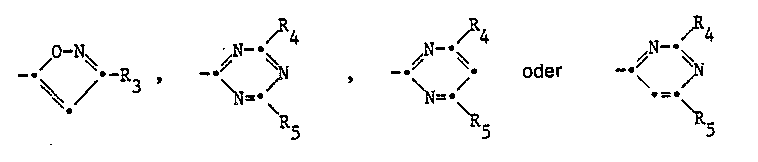

In diesen Formel bedeuten R₃, R₄ und Rₛ unabhängig voneinander Wasserstoff oder nicht-chromophore Substituenten. Beispiele für derartige Substituenten sind bereits weiter oben angeführt.

Von den erfindungsgemässen 4-Heterocyclylvinyl-4'-styryl-biphenylen sind jene der Formel (1) besonders zu erwähnen, worin R₁ Wasserstoff, Alkylsulfonyl, Phenylsulfonyl, Alkoxysulfonyl, Cyano, Trifluormethyl, die Sulfogruppe und deren Salze, die Carboxylgruppe und deren Salze oder eine Gruppe der Formel ―COOY₁, -CONY₁Y₂ oder ―SO₂N₁Y₂, worin Y₁ und Y₂ unabhängig voneinander für Wasserstoff, Alkenyl, Cycloalkyl, .unsubstituiertes oder nicht-chromophor substituiertes Alkyl, Phenylalkyl, oder Y₁ und Y₂ zusammen mit dem Stickstoffatom, an das sie gebunden sind, für einen 5- oder 6- gliedrigen gesättigten heterocyclischen Ring stehen, der gegebenenfalls noch zusätzlich 1 oder 2 Heteroatome als Ringglieder enthalten und gegebenenfalls durch Alkylgruppen substituiert sein kann, R₂ Wasserstoff, Chlor oder Alkyl, R₃ Wasserstoff, Alkyl, Cycloalkyl, Phenyl, Benzyl, Pyridyl oder Alkoxyalkyl und R₄ und R₅ unabhängig voneinander Wasserstoff, Alkyl, Phenyl, Alkoxy, Halogen, Amino, Mono- oder Dialkylamino, Alkylmercapto, Hydroxy oder Alkoxyalkyl bedeuten.

Bevorzugte nicht-chromophore Substituenten von Alkylgruppen sowie gesättigte Stickstoffheterocyclen (Y₁ + Y₂) sind weiter oben bereits aufgezählt.

Von besonderem Interesse sind jene erfindungsgemässen Verbindungen der Formel (1), worin R₁ Wasserstoff, C₁-C₄-Alkylsulfonyl, Phenylsulfonyl, C₁-C₄-Alkoxysulfonyl, Cyano, die Sulfogruppe und deren Salze, die Carboxylgruppe und deren Salze oder eine Gruppe der Formel ―COOY₁', ―CONY₁'Y₂' oder ―SO₂NY₁'Y₂', worin Y₁' und Y₂' unabhängig voneinander für Wasserstoff, C₁-C₄-Alkyl oder Benzyl stehen, R₂ Wasserstoff, Chlor oder C₁-C₄-Alkyl, R₃ Wasserstoff, C₁-C₄-Alkyl, Benzyl, Phenyl, Pyridyl oder C₂-C₈-Alkoxyalkyl und R₄ und R₅ unabhängig voneinander Wasserstoff, C₁-C₄-Alkyl, Phenyl, C₁-C₄-Alkoxy, Chlor, C₂-C₈-Alkoxyalkyl, C₁-C₄-Alkylmercapto, Amino, C₁-C₄-Alkylamino oder C₂-C₆-Dialkylamino bedeuten, und insbesondere solche Verbindungen der Formel (1), worin R₁ Wasserstoff, C₁-C₄-Alkylsulfonyl, Cyano, Carboxy, C₂-Cₛ-Alkoxycarbonyl oder Aminocarbonyl, R₂ Wasserstoff oder Chlor, R₃ Wasserstoff, C₁-C₄-Alkyl, Benzyl, Phenyl, Pyridyl oder C₂-C₆-Alkoxyalkyl und R₄ und Rₛ unabhängig voneinander Wasserstoff, C₁-C₄-Alkyl, Phenyl, C₁-C₄-Alkoxy, C₁-C4-Alkylmercapto, Amino, C₁-C₄-Alkyl- amino oder C₂-Cs-Dialkylamino bedeuten.

In besonders interessanten erfindungsgemässen 4-Heterocyclylvinyl-4'-styryl-biphenylen stellt der Substituent A einen unsubstituierten oder substituierten (Substitution wie vorstehend definiert) 1,3,4-Oxadiazol-2-yl-, 1,2,4-Oxadiazol-5-yl-, 1,2,4-Oxadiazol-3-yl-, 1,2-Oxazol-3-yl-, 1,2-Oxazol-5-yl-, oder 1,3-Pyrimidin-2-ylrest, vorzugsweise einen 1,3,4-Oxadiazol-2-yl-, 1,2,4-Oxadiazol-5-yl- oder 1,2,4-Oxadiazol-3- ylrest dar.

Bevorzugte erfindungsgemässe 4-Heterocyclylvinyl-4'-styryl-biphenyle entsprechen der Formel 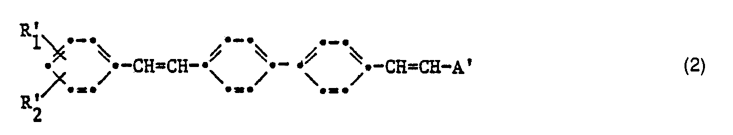 worin R₁' C₁-C₄-Alkylsulfonyl, Cyano, Carboxy oder C₂-Cₛ-Alkoxy-carbonyl, R₂' Wasserstoff oder Chlor und A' einen Rest der Formel 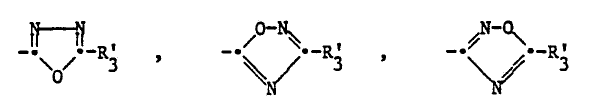 bedeuten, worin R₃' für Wasserstoff, C₁-C₄-Alkyl oder Phenyl, vorzugsweise für C₁-C₄-Alkyl steht.

Von allen vorgenannten erfindungsgemässen 4-Heterocyclylvinyl-4'-styryl-biphenylen sind jene von ganz besonderem praktischem Interesse, in denen der Substituent R₁ bzw. R₁' für Cyano, C₁-C₄-Alkylsulfonyl oder C₂-C₅-Alkoxycarbonyl, vorzugsweise aber für Cyano, und R₂ bzw. R₂' für Wasserstoff steht.

Die erfindungsgemässen 4-Heterocyclylvinyl-4'-styryl-biphenyle der Formel (1) und damit auch die darunter fallenden bevorzugten Verbindungen können nach verschiedenen an sich bekannten Verfahren erhalten werden.

Ein Verfahren zur Herstellung der Verbindungen der Formel (1) besteht darin, dass man eine Verbindung der Formel 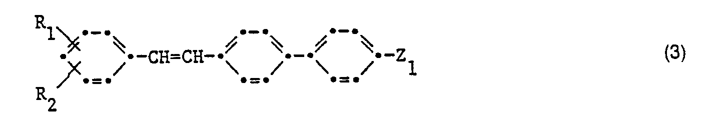 mit einer Verbindung der Formel Z₂―A, oder eine Verbindung der Formel 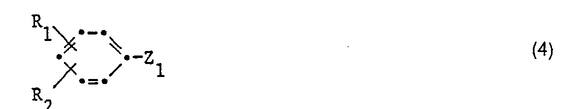 mit einer Verbindung der Formel 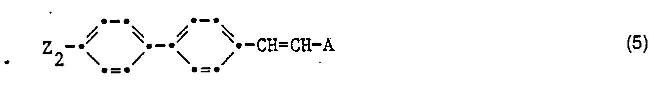 umsetzt, wobei in obigen Formeln R_{1'} R₂ und A wie in Formel (1) definiert sind und jeweils einer der beiden Substituenten Z₁ und Z₂ die Gruppe 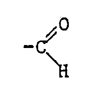 und der andere Methyl, Carboxymethyl (oder ein funktionelles Derivat davon) oder eine Gruppe der Formel 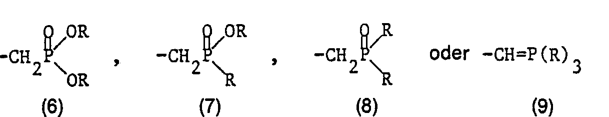 bedeutet, worin R unsubstituiertes oder substituiertes C₁-C₅-Alkyl oder unsubstituiertes oder substituiertes Phenyl bedeutet.

Als funktionelle Derivate der Carboxymethylgruppe kommen z. B. die Gruppen der Formeln -CH₂CN, -CH₂CONH₂, --CH₂COCI oder -CH₂COOR' in Frage, wobei R' C₁-C₄-Alkyl bedeutet. Werden funktionelle Derivate der Carboxymethylgruppe eingesetzt, kann es je nach Reaktionsbedingungen erforderlich sein, nach der Hauptreaktion einen Verseifungsschritt mit Decarboxylierung anzuschliessen.

Die Kondensation einer Verbindung der Formel (3) mit einer solchen der Formel Z₂A bzw. einer Verbindung der Formel (4) mit einer solchen der Formel (5) kann allgemein in der Schmelze, vorzugsweise jedoch in einem indifferenten Lösungsmittel bei Temperaturen zwischen 20 und 150 °C, nötigenfalls in Gegenwart eines Katalysators durchgeführt werden. Als Lösungmittel kommen beispielsweise Kohlenwasserstoffe, wie Toluol und Xylol oder Alkohole wie Methanol, Aethanol, Isopropanol, Butanol, Glykol, Glykoläther wie 2-Methoxyäthanol, Hexanole, Cyclohexanol und Cyclooctanol, ferner Aether wie Diisopropyläther, Tetrahydrofuran und Dioxan in Betracht. Besonders geeignet sind polare organische Lösungsmittel wie Dimethylformamid, N-Methylpyrrolidon und Dimethylsulfoxid. Auch in wässriger Lösung lassen sich einige der Umsetzungen durchführen. Als Katalysatoren eignen sich beispielsweise tertiäre Amine wie Pyridin, Picolin, Triäthylamin sowie Piperidin, Zinkchlorid, Borsäure, Borsäureanhydrid, Essigsäureanhydrid, p-Toluolsulfonsäure, ferner Alkaliacetate, Alkali- oder Erdalkalihydroxide, Alkalialkoholate, Phthalimidkalium und Kaliumcarbonat.

Ist einer der Substituenten Z₁ oder Z₂ eine Methylgruppe oder eine Carboxymethylgruppe oder ein Derivat davon, kann die Kondensation z. B. auch wie in der DE-A-2 262 340 oder der US-A-3 728 399 beschrieben durchgeführt werden. Sie wird demnach zweckmässig in Gegenwart basischer Katalysatoren unter den Bedingungen der Knoevenagel-Reaktion ausgeführt. Da die Reaktivitäten sowohl der Aldehydgruppe als auch der Methyl- bzw. Methylengruppe in Abhängigkeit von den jeweiligen Substituenten unterschiedlich sind, müssen die Reaktionsbedingungen entsprechend angepasst werden. Man arbeitet bei Temperaturen im Bereich von 75-200 °C ohne oder mit Lösungsmitteln, die, falls gewünscht, ein azeotropes Entfernen des Reaktionswassers ermöglichen. Es kommen z. B. in Betracht : Benzol, Chlorbenzol, Toluol, Xylol, Pyridin, Essigsäure, DMF. Geeignete basische Katalysatoren sind u. a. Benzolsulfonamidnatrium, Carbazol-Kalium, Ammoniumacetat, insbesondere jedoch organische Stickstoffbasen wie Dibutylamin, Dibenzylamin, Morpholin, Hexamethylenimin und vorzugsweise Piperidin. Die Umsetzung ist beendet, wenn die CO₂-Entwicklung aufhört.

Ist einer der Substituenten Z₁ oder Z₂ eine phosphorhaltige Gruppe der Formeln (6)-(9), erfolgt die Umsetzung zweckmässig nach den Bedingungen der Wittig-Horner-Synthese. Bei dieser Synthese verwendet man als Lösungsmittel vorteilhaft indifferente Lösungsmittel, beispielsweise Kohlenwasserstoffe wie Toluol oder Xylol oder Alkohole wie Methanol, Aethanol, Isopropanol, Butanol, Glykol, Glykoläther wie 2-Methoxyäthanol, Hexanol, Cyclohexanol, Cyclooctanol, ferner Aether wie Diisopropyläther, Dioxan, Tetrahydrofuran, weiterhin Formamide oder N-Methylpyrrolidon. Besonders geeignet sind dipolare organische Lösungsmittel wie Dimethylformamid und Dimethylsulfoxid.

Vorzugsweise werden Kondensationsmittel eingesetzt. Als solche kommen z. B. stark basische Verbindungen in Betracht, wie Alkali- und Erdalkalimetallhydroxide, Alkali- und Erdalkaliamide und Alkali- und Erdalkalimetallalkoholate, beispielsweise Kaliumhydroxid, Natriumhydroxid, Kalium-tert.-butylat, Natriumamid oder Natriummethylat, ferner die Alkaliverbindungen des Dimethylsulfoxids und Alkalihydride sowie gegebenenfalls Alkalimetalldispersionen.

Man arbeitet vorzugsweise im Temperaturbereich von 0 bis 100 °C. Die erfindungsgemässen Verbindungen werden ebenfalls erhalten, wenn man anstelle von Phosphonoverbindungen die entsprechenden quartären Phosphonium-Salze, beispielsweise die Triphenylphosphoniumsalze, einsetzt und diese über die Stufe der Phosphorylene mit den Aldehyden kondensiert.

An den Reaktionsprodukten der vorstehenden Verfahren können noch weitere an sich bekannte Umwandlungen vorgenommen werden wie funktionelle Abwandlungen von Carboxyl- und Sulfogruppen z. B. Umesterungen oder Austausch von Halogenatomen gegen Cyangruppen. Von besonderer Bedeutung ist die Ueberführung von Carbonsäureestern in Carbonamide durch Kondensation mit Ammoniak oder Aminen.

Die Aldehyde der Formeln (3) bis (5) und Z₂-A (Z_{1'} Z₂ = CHO) sind bekannt oder können nach bekannten Verfahren leicht erhalten werden. Etliche dieser Aldehyde, insbesondere der Formel (3), sind aus der EP-A 54 511 bekannt. Dort nicht beschriebene Aldehyde können nach den dort oder in den Beispielen dieser Anmeldung angegebenen Angaben auf analoge Weise erhalten werden. Aldehyde, worin R₁ = Carboxyl, erhält man auch durch, vorzugsweise saure, Hydrolyse der entsprechenden Carbonsäureester.

Die Phosphorverbindungen (Zi, Z₂ = Gruppen der Formeln (6)-(9)), insbesondere jene der Formel Zᵣ-A sind z. B. aus der DE-A-3 006 351 bekannt oder können nach den dort angegebenen Verfahren leicht erhalten werden. Siehe auch EP-A-31 796.

Ein weiteres Verfahren zur Herstellung von Verbindungen der Formel (1), worin A eine Gruppe der Formel 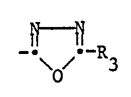 ist und R₃ für Wasserstoff oder einen nicht-chromophoren Substituenten steht, besteht darin, dass man eine Carbonsäure der Formel 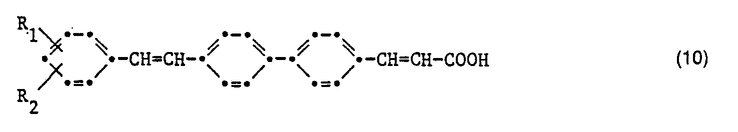 oder ein funktionelles Derivat dieser Säure mit einem Carbonsäure-hydrazid der Formel H₂N―NH―CO―R₃, oder eine Verbindung der Formel 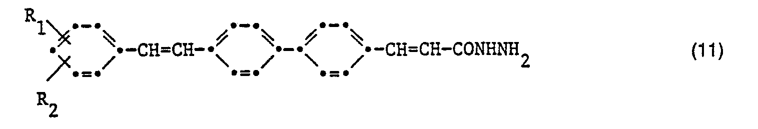 mit einer Carbonsäure der Formel HOOC-R₃ oder einem funktionellen Derivat dieser Säure umsetzt, wobei R₁ und R₂ wie in Formel (1) und R₃ wie oben definiert sind, und das erhaltene Kondensationsprodukt in Gegenwart eines wasserabspaltenden Mittels cyclisiert.

Als funktionelle Derivate der Carbonsäuren können beispielsweise Carbonsäurehalogenide wie Chloride und Bromide, Carbonsäureanhydride, Carbonsäureester wie Carbonsäurealkylester mit 1-4 C-Atomen in der Alkylgruppe, Carbonsäureamide und Nitrile eingesetzt werden. Bevorzugt sind die Carbonsäurechloride.

Bei der Umsetzung entsteht in erster Stufe eine Hydrazinverbindung der Formel

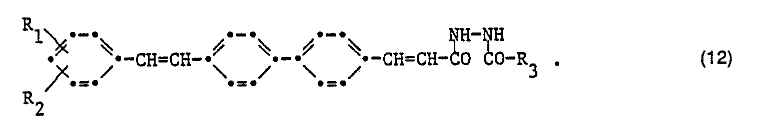

Diese Verbindung kann isoliert werden oder die zweite Stufe (Cyclisierung) kann ohne deren Isolierung direkt angeschlossen werden.

Die Umsetzung der Säure (10) mit dem Hydrazid NH₂NH-CO-R₃ bzw. der Säure HOOC-R₃ mit dem Hydrazid (11) kann durch Erhitzen auf Temperaturen über 100 °C erfolgen, zweckmässig auf 120 bis 300 °C, vorteilhaft in Gegenwart eines inerten organischen Lösungsmittels wie Toluol, Xylolen, Chlorbenzol, Dichlorbenzolen, Trichlorbenzol oder Nitrobenzol oder falls Säurechloride verwendet werden, vorzugsweise in Gegenwart eines katalytisch wirkenden oder säurebindenden Mittels z. B. in Pyridinbasen, wie Picolinen oder Pyridin, weiteren tertiären Aminen wie Triäthylamin oder Alkalihydroxiden bei 0-120 °C. Die Ueberführung in die Oxadiazolylverbindung (Cyclisierung) geschieht in der Regel durch Behandlung der Diacylhydrazinverbindung der Formel (12) mit waserabspaltenden Mitteln, wie Phosphoroxychlorid, Phosphortrichlorid, Phosphorpentachlorid, Phosphorpentoxid, Polyphosphorsäure, Schwefelsäure, Sulfurylchlorid, Oleum-Dimethylformamid, Zinkchlorid, Aluminiumchlorid, p-Toluolsulfonsäure oder Thionylchlorid, bei Temperaturen zwischen 100 und 250 °C. Gewünschtenfalls können auch hochsiedende organische Lösungsmittel wie beispielsweise Dimethylformamid, Dichlorbenzol, Trichlorbenzol, Nitrobenzol, Pyridin und aliphatische, gegebenenfalls verätherte Oxyverbindungen, z. B. Propylenglykol, Aethylenglykolmonoäthyläther, Diäthylenglykoldiäthyläther oder Diäthylenglykol-dibutyläther mitverwendet werden.

Eine besonders vorteilhafte Ausführungsform besteht darin, dass man ein Säurehalogenid, vorzugsweise-chlorid, der Säure der Formel (10) mit einem Hydrazid der Formel H₂NNH--CO-R₃ vorzugsweise in Gegenwart eines katalytisch wirksamen oder Halogenwasserstoff bindenden Mittels umsetzt und die erhaltene Acylhydrazinverbindung einer Oxadiazol-Ringschlussreaktion durch Behandlung mit Wasserabspaltungsmittel, z. B. Phosphoroxychlorid, zwischen 100 und 200 °C unterwirft.

Zur Herstellung von erfindungsgemässen Verbindungen der Formel (1), worin A eine Gruppe der Formel 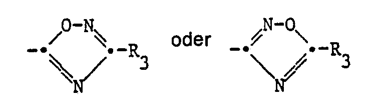 (mit R₃ = Wasserstoff oder nichtchromophorer Substituent) bedeutet, kann man auch so verfahren, dass man eine Carbonsäure der Formel 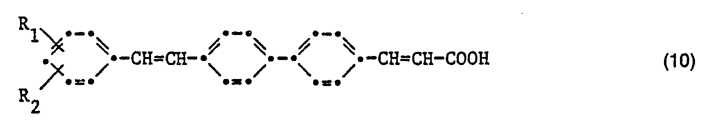 oder ein funktionelles Derivat dieser Säure mit einem Amidoxim der Formel 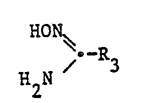 oder eine Verbindung der Formel 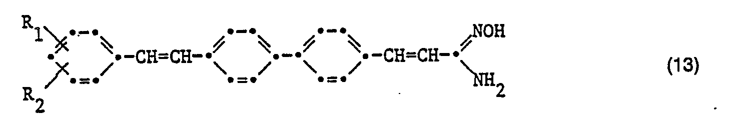 mit einer Carbonsäure der Formel HOOC-R₃ oder einem funktionellen Derivat dieser Säure umsetzt, wobei R₁ und R₂ wie in Formel (1) und R₃ wie oben definiert ist, und das erhaltene Kondensationsprodukt in Gegenwart eines wasserabspaltenden Mittels cyclisiert.

Als funktionelle Derivate der Carbonsäuren können beispielsweise Carbonsäurehalogenide wie Chloride und Bromide, Carbonsäureanhydride, Carbonsäureester wie Carbonsäurealkylester mit 1-4 C-Atomen in der Alkylgruppe, Carbonsäureamide und Nitrile eingesetzt werden. Bevorzugt sind die Carbonsäurechloride.

Die erste Stufe (Umsetzung einer Säure oder eines Derivates davon mit einem Amidoxim) kann z. B. in Gegenwart eines Säureacceptors (wenn z. B. ein Carbonsäurehalogenid verwendet wird) durchgeführt werden. Bevorzugte Umsetzungstemperaturen liegen bei 0 bis 80 °C. Die gebildeten Acylierungsprodukte können isoliert werden ; vorzugsweise wird aber die Cyclisierung ohne Isolierung des Zwischenproduktes durchgeführt. Die Cyclisierung kann z. B. durch Erhitzen auf 100-200 °C, vorzugsweise 160-200 °C erfolgen, vorzugsweise unter Entfernung des gebildeten Wassers durch azeotrope Destillation mit einem geeigneten Lösungsmittel.

Als Lösungsmittel für die Reaktion eignen sich z. B. Toluol, Xylol, Aethylenchlorid, Chlorbenzol, Di-oder Trichlorbenzol und insbesondere Dimethylformamid, N-Methylpyrrolidon, Dimethylsulfoxid oder Nitrobenzol. Säurebindende Mittel sind beispielsweise Na₂C0₃, K₂C0₃, CaC0₃ oder tert. Amine, beispielsweise Pyridin, Triäthylamin oder N,N-Dimethylanilin.

Die in den beiden letztgenannten Verfahren benötigten Ausgangsprodukte (Verbindungen der Formeln (10), (11), (13), H₂NNH--CO-R₃, HOOC-R₃ und 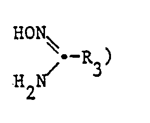 sind bekannt oder können nach an sich bekannten Verfahren hergestellt werden. Siehe dazu EP-A 6 171, EP-A 22 491, EP-A 72 905 und DE-A 2 350 570.

Die erfindungsgemässen 4-Heterocyclylvinyl-4'-styryl-biphenyle der Formel (1) zeigen in gelöstem oder feinverteiltem Zustand eine ausgeprägte Fluoreszenz. Sie können zum optischen Aufhellen der verschiedensten synthetischen, halbsynthetischen oder natürlichen hochmolekularen organischen Materialien verwendet werden. Diese Verwendung ist ebenfalls Gegenstand der vorliegenden Erfindung.

Als aufzuhellende Materialien seien beispielsweise, ohne dass durch die nachfolgende Uebersicht irgendeine Beschränkung hierauf ausgedrückt werden soll, die folgenden Gruppen von organischen Materialien, soweit eine optische Aufhellung derselben in Betracht kommt, genannt :
I. Synthetische organische hochmolekulare Materialien :
   a) Polymerisationsprodukte auf Basis mindestens eine polymerisierbare Kohlenstoff-Kohlenstoff-Doppelbindung enthaltender organischer Verbindungen, d. h. deren Homo- oder Copolymerisate sowie deren Nachbehandlungsprodukte wie beispielsweise Vernetzungs-, Pfropfungs- oder Abbauprodukte, Polymerisat-Verschnitte oder durch Modifizierung reaktionsfähiger Gruppen erhaltene Produkte, beispielsweise Polymerisate auf Basis von a,ß-ungesättigten Carbonsäuren oder Derivaten solcher Carbonsäuren, insbesondere von Acrylverbindungen (wie z. B. Acrylestern, Acrylsäure, Acrylnitril, Acrylamiden und deren Derivaten oder deren Methacryl-Analoga), von Olefin-Kohlenwasserstoffen (wie z. B. Aethylen, Propylen, Styrole oder Diene, ferner sogenannte ABS-Polymerisate), Polymerisate auf Basis von Vinyl-und Vinyliden-Verbindungen (wie z. B. Vinylchlorid, Vinylalkohol, Vinylidenchlorid),
   b) Polymerisationsprodukte, die durch Ringöffnung erhältlich sind, z. B. Polyamide vom Polycaprolactam-Typ, ferner Polymere, die sowohl über Polyaddition als auch Polykondensation erhältlich sind, wie Polyäther oder Polyacetale,
   c) Polykondensationsprodukte oder Vorkondensate auf Basis bi- oder polyfunktioneller Verbindungen mit kondensationsfähigen Gruppen, deren Homo- und Mischkondensationsprodukte sowie Produkte der Nachbehandlung, wie beispielsweise Polyester, insbesondere gesättigte (z. B. Aethylenglykolterephthalsäure-Polyester) oder ungesättigte (z. B. Maleinsäure-Dialkohol-Polykondensate sowie deren Vernetzungsprodukte mit anpolymerisierbaren Vinylmonomeren), unverzweigte sowie verzweigte (auch auf Basis höherwertiger Alkohole, wie z. B. Alkydharze) Polyester, Polyamide (z. B. Hexamethylendiamin-adipat), Maleinatharze, Melaminharze, deren Vorkondensate und Analoga, Polycarbonate, Silikone,
   d) Polyadditionsprodukte wie Polyurethane (vernetzt und unvernetzt), Epoxidharze.
11. Halbsynthetische organische Materialien, z. B. Celluloseester verschiedener Veresterungsgrade (sogenanntes 2 1/2-Acetat, Triacetat) oder Celluloseäther, regenerierte Cellulose (Viskose, Kupferammoniak-Cellulose) oder deren Nachbehandlungsprodukte, Casein-Kunststoffe.
111. Natürliche organische Materialien animalischen oder vegetabilischen Ursprungs, beispielsweise auf Basis von Cellulose oder Proteinen wie Baumwolle, Wolle, Leinen, Seide, natürliche Lackharze, Stärke, Casein.

Die optisch aufzuhellenden organischen Materialien können den verschiedenartigsten Verarbeitungszuständen (Rohstoffe, Halbfabrikate oder Fertigfabrikate) angehören. Sie können andererseits in Form der verschiedenartigsten geformten Gebilde vorliegen, d. h. beispielsweise als vorwiegend dreidimensional ausgedehnte Körper wie Platten, Profile, Spritzgussformlinge, verschiedenartige Werkstücke, Schnitzel, Granulate oder Schaumstoffe, ferner als vorwiegend zweidimensional ausgebildete Körper wie Filme, Folien, Lacke, Ueberzüge, Imprägnierungen und Beschichtungen oder als vorwiegend eindimensional ausgebildete Körper wie Fäden, Fasern, Flocken, Drähte. Die besagten Materialien können andererseits auch in ungeformten Zuständen in den verschiedenartigsten homogenen oder inhomogenen Verteilungsformen, wie z. B. als Pulver, Lösungen, Emulsionen, Dispersionen, Latices, Pasten oder Wachse vorliegen.

Fasermaterialien können beispielsweise als endlose Fäden (verstreckt oder unverstreckt), Stapelfasern, Flocken, Strangware, textile Fäden, Garne, Zwirne, Faservliese, Filze, Watten, Beflockungs-Gebilde oder als textile Gewebe oder textile Verbundstoffe, Gewirke sowie als Papiere, Pappen oder Papiermassen vorliegen.

Den erfindungsgemäss anzuwendenden Verbindungen der Formel (1) kommt vor allem Bedeutung für die Behandlung von textilen organischen Materialien, insbesondere textilen Geweben, zu. Sofern Fasern, welche als Stapelfasern oder Endlosfäden, in Form von Strangen, Geweben, Gewirken, Vliesen beflockten Substraten oder Verbundstoffen vorliegen können, erfindungsgemäss optisch aufzuhellen sind, geschieht dies mit Vorteil in wässerigem Medium, worin die betreffenden Verbindungen in feinverteilter Form (Suspensionen, sogenannten Mikrodispersionen, gegebenenfalls Lösungen) vorliegen. Gegebenenfalls können bei der Behandlung Dispergier-, Stabilisier-, Netz- und weitere Hilfsmittel zugesetzt werden.

Bei der Applikation kann man in neutralem, alkalischem oder saurem Baae arbeiten. Die Behandlung wird üblicherweise bei Temperaturen von etwa 20 bis 140 °C, beispielsweise bei Siedetemperatur des Bades oder in deren Nähe (etwa 90 °C), durchgeführt. Für die erfindungsgemässe Veredlung textiler Substrate kommen auch Lösungen oder Emulsionen in organischen Lösungsmitteln in Betracht, wie dies in der Färbereipraxis in der sogenannten Lösungsmittelfärberei (Foulard-Thermofixierapplikation, Ausziehfärbeverfahren in Färbemaschinen) praktiziert wird.

Die optischen Aufheller gemäss vorliegender Erfindung können ferner den Materialien vor oder während deren Verformung zugesetzt bzw. einverleibt werden. So kann man sie beispielsweise bei der Herstellung von Filmen, Folien (z. B. Einwalzen in Polyvinylchlorid in der Hitze) oder Formkörpern der Pressmasse oder Spritzgussmasse beifügen.

Sofern die Formgebung voll- oder Halbsynthetischer organischer Materialien durch Spinnverfahren bzw. über Spinnmassen erfolgt, können die optischen Aufheller nach folgenden Verfahren appliziert werden :
- Zugabe zu den Ausgangssubstanzen (z. B. Monomeren) oder Zwischenprodukten (z. B. Vorkondensaten, Praepolymeren), d. h. vor oder während der Polymerisation, Polykondensation oder Polyaddition,
- Aufpudern auf Polymerisatschnitzel oder Granulate für Spinnmassen,
- Badfärbung von Polymerisatschnitzeln oder Granulaten für Spinnmassen,
- Dosierte Zugabe zu Spinnschmelzen oder Spinnlösungen,
- Applikation auf Spinnkabel vor dem Verstrecken.

Die erfindungsgemässen optischen Aufheller können beispielsweise auch in folgenden Anwendungsformen eingesetzt werden:
a) In Mischungen mit Farbstoffen (Nuancierung) oder Pigmenten (Farb- oder insbesondere z. B. Weisspigmenten) oder als Zusatz zu Färbebädern, Druck-, Aetz- oder Reservepasten, ferner auch zur Nachbehandlung von Färbungen, Drucken oder Aetzdrucken,
b) in Mischungen mit sogenannten «Carriern», Netzmitteln, Weichmachern, Quellmitteln, Antioxydantien, Lichtschutzmitteln, Hitzestabilisatoren, chemischen Bleichmitteln (Chlorit-Bleiche, Bleichbäder-Zusätze),
c) in Mischung mit Vernetzern, Appreturmitteln (z. B. Stärke oder synthetischen Appreturen) sowie in Kombination mit den verschiedensten Textilveredlungsverfahren, insbesondere Kunstharzausrüstungen (z. B. Knitterfest-Ausrüstungen wie «wash-and-wear», «permanent-pᵣₑₛₛ», «non-iron»), ferner Flammfest-, Weichgriff-, Schmutzablöse («anti-soiling»)- oder Antistatisch-Ausrüstungen oder antimikrobiellen Ausrüstungen,
d) Einarbeiten der optischen Aufhellmittel in polymere Trägermaterialien (Polymerisations-, Polykondensations- oder Polyadditionsprodukte) in gelöster oder dispergierter Form für Anwendung z. B. in Beschichtungs-, Imprägnier- oder Bindemitteln (Lösungen, Dispersionen, Emulsionen) für Textilien, Vliese, Papier, Leder,
e) als Zusätze zu den verschiedenstan industriellen Produkten, um dieselben marktfähiger zu machen (z. B. Aspektverbesserung von Seifen, Waschmitteln, Pigmenten),
f) in Spinnbadpräparationen, d. h. als Zusätze zu Spinnbädern, wie sie zur Gleitfähigkeitsverbesserung für die Weiterverarbeitung von Synthese-Fasern verwendet werden, oder aus einem speziellen Bad vor der Verstreckung der Faser,
g) in Mitteln zum optischen Aufhellen von hochmolekularen organischen Materialien der vorstehend angegebenen Zusammensetzungen, welche Mittel gegebenenfalls übliche Formulierungszusätze und/ oder gegebenenfalls weitere optische Aufheller aus anderen Aufhellerklassen enthalten,
h) in Kombination mit anderen, optisch aufhellend wirkenden Substanzen; ferner als Zusätze zu sogenannten «master batches»,
i) als Scintillatoren, für verschiedene Zwecke photographischer Art, wie z. B. für elektrophotographische Reproduktion und Supersensibilisierung,
j) je nach Substitution als Laser-Farbstoffe.

Mittel, die die erfindungsgemässen optischen Aufheller der Formel (1) enthalten, sind ebenfalls Gegenstand der Erfindung.

Als übliche Formulierungszusätze, die in solchen Mitteln enthalten sein können, kommen z. B. verschiedenste Hilfs- und Coupiermittel, wie z. B. wasserfreies Natriumsulfat, Natriumsulfat-decahydrat, Natriumchlorid, Natriumcarbonat, Alkalimetallphosphate, wie Natrium- oder Kaliumorthophosphat, Natrium- oder Kaliumpyrophosphat und Natrium- oder Kaliumtripolyphosphate oder Alkalimetallsilicate in Betracht. Aber auch wässrige Formulierungen fallen unter die erfindungsgemässen Mittel, z. B. auch die Applikationslösungen, mit denen Textilfasern optisch aufgehellt werden und die üblichen Zusätze enthalten.

Gegenstand der Erfindung sind auch Mittel, die neben einem oder mehreren Verbindungen der Formel (1) noch einen oder mehrere optische Aufheller aus der Klasse der Triazinyl-pyrene, 2-Styrylbenzoxazole, 1,4-Bis-styrylbenzole, 4-Benzoxazolylstilbene, 4,4'-Divinylstilbene, Naphthalimide, 4,4'-Bis-styrylbiphenyle, 4,4'-Bis-triazolyl-stilbene, Bis-benzoxazolyl-thiophene, -naphthaline oder -äthyiene, Oxadiazolyl-stilbene, Naphthotriazol-2-yl-stilbene (z. B. bekannt aus den deutschen Offerilegungsschriften 25 39 537 und 25 39 461) oder der Cumarine, z. B. der Triazolylcumarine (bekannt aus den Schweizer Patentschriften 566.359 und 592.189) und der Pyrazolylcumarine, enthalten oder aus solchen Aufhellerkombinationen bestehen. Bevorzugt sind solche Mittel, die als Aufheller-Aktivsubstanz 10-99 %, insbesondere 30-70%, eines erfindungsgemässen optischen Aufhellers der Formel (1) und 90-1 %, insbesondere 70-30 %, eines optischen Aufhellers aus den oben genannten Klassen enthalten. Derartige Kombinationen von optischen Aufhellern müssen keine weiteren Zusätze enthalten, können also auch als reine Aufhellermischungen vorliegen. Bevorzugt sind die Aufheller aus den obengenannten Klassen in den erfindungsgemässen Aufhellerkombinationen Polyesteraufheller.

Derartige Kombinationen haben den Vorteil, dass dadurch auf Textilfasern, vor allem auf Polyesterfasern ein besonders schöner neutraler Weisston von hoher Brillanz erzielt werden kann.

Praktisch besonders wichtige optische Aufheller, die zusammen mit den erfindungsgemässen Verbindungen der Formel (1) in den oben genannten Aufhellerkombinationen eingesetzt werden können, sind unter anderen:

4-Chlor-2'-cyano-4'-(naphthotriazol-2-yl) stilben, 3-Phenyl-7-(3'-methylpyrazol-1-yl)cumarin, 4-(Pyrimidin-2-yl)-4'-(cyanovinyl)stilben, 1-(4'-Cyanophenyl)-2-(5'-6'-dimethylbenzoxazol-2'-yl)äthylen, 3-Phenyl-7-(4'-phenyl-5'-methyl-v-triazol-2-yl)-cumarin, 3-(4'-Chlorpyrazol-1'-yl)-7-(4'-phenyl-5'-methyl-v-triazol-2-yl)cumarin, 1-Methyl-5,6-diäthoxynaphthalimid, 1-Methyl-5-methoxynapthalimid, 3-Phenyl-7-naphthotria- zol-2'-yl-cumarin und insbesondere 1,4-Bis-(benzoxazol-2-yl)-naphthalin, 1-(4'-Methoxycarbonylphenyl)-2-(5',6'-dimethylbenzoxazol-2'-yl)äthylen, 4,4'-Bis-(äthoxycarbonyl-vinyl)-stilben, 4,4'-Bis-(cyano-vinyl)-stilben, 1,4-Bis-(2'-cyanostyryl)-benzol, 2,5-Bis-(benzoxazol-2'-yl)-thiophen, - 4-Phenyl-4'-(5",7"-dimethyl- benzoxazol-2"-yl)-stilben, 1,2-Bis-(5"-methyl-benzoxazol-2'-yl)-äthylen, 4-(Benzoxazol-2'-yl)-4'-(3"-methyl-1",2",4"-oxadiazol-5"-yl)-stilben oder 2,4-Dimethoxytriazin-6-yl-pyren.

Das Mengenverhältnis zwischen den vorerwähnten Aufhellern und dem jeweiligen 4-Heterocyclylvi- nyl-4'-styryl-biphenyl der Formel (1) reicht in besonders bevorzugten Aufhellermischungen von 1 : 2 bis 2: 1.

Besonders bevorzugt werden mit den erfindungsgemässen optischen Aufhellern, die keine Sulfogruppen enthalten, Substrate aus Polyester aufgehellt, insbesondere Textilmaterialien aus Polyester. Aber auch Polyamid und andere Kunstfasern können sehr gut aufgehellt werden. Sulfogruppenhaltige Verbindungen der Formel (1) können auch zum optischen Aufhellen von Cellulose, insbesondere Baumwolltextilien verwendet werden. Mischungen von sulfogruppenhaltigen und nicht sulfogruppenhaltigen Verbindungen der Formel (1) eignen sich somit auch zum Aufhellen von Polyester/Baumwolle-Mischfasern.

Wird das Aufhellverfahren mit Textilbehandlungs- oder Veredlungsmethoden kombiniert, so kann die kombinierte Behandlung in vielen Fällen vorteilhafterweise mit Hilfe entsprechender beständiger Präparate erfolgen, welche die optisch aufhellenden Verbindungen in solcher Konzentration enthalten, dass der gewünschte Aufhelleffekt erreicht wird.

In gewissen Fällen werden die Aufheller durch eine Nachbehandlung zur vollen Wirkung gebracht. Diese kann beispielsweise eine chemische (z. B. Säure-Behandlung), eine thermische oder eine kombinierte chemisch/thermische Behandlung darstellen. So verfährt man beispielsweise bei einer optischen Aufhellung einer Reihe von Fasersubstraten, z. B. von Polyesterfasern, mit den erfindungsgemässen Aufhellern zweckmässig in der Weise, dass man diese Fasern mit den wässerigen Dispersionen (gegebenenfalls auch Lösungen) der Aufhellmittel bei Temperaturen unter 75 °C, z. B. bei Raumtemperatur, imprägniert und einer trockenen Wärmebehandlung bei Temperaturen über 100 °C unterwirft, wobei es sich im allgemeinen empfiehlt, dass Fasermaterial vorher noch bei mässig erhöhter Temperatur, z. B. bei mindestens 60 °C bis etwa 130 °C zu trocknen. Die Wärmebehandlung in trockenem Zustande erfolgt dann vorteilhaft bei Temperaturen zwischen 120 und 225 °C, beispielsweise durch Erwärmen in einer Trockenkammer, durch Bügeln im angegebenen Temperaturintervall oder auch durch Behandeln mit trockenem, überhitztem Wasserdampf. Die Trocknung und trockene Wärmebehandlung können auch .unmittelbar nacheinander ausgeführt oder in einen einzigen Arbeitsgang zusammengelegt werden.

Die Menge der erfindungsgemäss zu verwendenden optischen Aufheller, bezogen auf das optisch aufzuhellende Material, kann in weiten Grenzen schwanken. Schon mit sehr geringen Mengen, in gewissen Fällen z. B. solchen von 0,001 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,01 und 0,5 Gewichtsprozent von Interesse.

Besonders bevorzugte Anwendungsgebiete für die erfindungsgemässen Verbindungen sind die folgenden : Optisches Aufhellen von Polyester, und zwar sowohl von Polyesterfasern und -gewebe nach dem Auszieh- oder Foulardthermverfahren, als auch von Polyesterspinnmassen. Mischgewebe aus Polyester und Baumwolle oder Wolle werden ebenfalls sehr vorteilhaft mit Hilfe der erfindungsgemässen Verbindungen aufgehellt. Beispiele für weitere Substrate, die vorteilhaft mit Hilfe der Verbindungen der Formel (1) aufgehellt werden können, sind: Polyamidfasergewebe, Celluloseacetatgewebe sowie Polystyrol- und Polyvinylchloridmassen. Besonders bevorzugt ist jedoch die Verwendung zum optischen Aufhellen von Polyesterfasern nach dem Auszieh- und Foulardthermverfahren.

Die erfindungsgemässen Verbindungen der Formel (1), insbesondere jene, die Sulfogruppen als Substituenten enthalten, eignen sich auch besonders als Zusätze für Waschbäder oder zu Gewerbe- und Haushaltwaschmitteln, wobei sie in verschiedener Weise zugesetzt werden können. Zu Waschbädern werden sie zweckmässig in Form ihrer Lösungen in Wasser oder organischen Lösungsmitteln oder auch in feiner Verteilung als wässrige Dispersionen zugegeben. Zu Haushalt- oder gewerblichen Waschmittel werden sie vorteilhaft in irgendeiner Phase des Herstellungsprozesses der Waschmittel, z. B. der sogenannten «slurry• vor dem Zerstäuben dem Waschpulver oder bei der Vorbereitung flüssiger Waschmittelkombinationen, zugesetzt. Die Zugabe kann sowohl in Form einer Lösung oder Dispersion in Wasser oder anderen Lösungsmitteln als auch ohne Hilfsmittel als trockenes Aufhellerpulver erfolgen. Man kann die Aufhellmittel beispielsweise mit den waschaktiven Substanzen vermischen, verkneten oder vermahlen und so dem fertigen Waschpulver zumischen. Sie können jedoch auch gelöst oder vordispergiert auf das fertige Waschmittel aufgesprüht werden.

Als Waschmittel kommen die bekannten Mischungen von Waschaktivsubstanzen wie beispielsweise Seifen in Form von Schnitzeln und Pulver, Synthetika, lösliche Salze von Sulfonsäurehalbestern höherer Fettalkohole, höher und/oder mehrfach alkylsubstituierten Arylsulfonsäuren, Sulfocarbonsäureester mittlerer bis höherer Alkohole, Fettsäureacylaminoalkyl- oder -aminoarylglycerinsulfonate, Phosphorsäureester von Fettalkoholen usw. in Frage. Als Aufbaustoffe, sogenannte « Builders », kommen z. B. Alkalipoly- und -polymetaphosphate, Alkalipyrophosphate, Alkalisalze der Carboxymethylcellulose und andere « Soilredepositionsinhibitoren •, ferner Alkalisilikate, Alkalicarbonate, Alkaliborate, Alkaliperborate, Nitrilotriessigsäure, Aethylendiamino-tetraessigsäure, Schaumstabilisatoren wie Alkanolamide höherer Fettsäuren, in Betracht. Ferner können in den Waschmitteln beispielsweise enthalten sein: antistatische Mittel, rückfettende Hautschutzmittel wie Lanolin, Enzyme, Antimikrobika, Parfüme und Farbstoffe.

Die erfindungsgemässen Verbindungen haben den besonderen Vorteil, dass sie auch bei Gegenwart von Aktivchlorspendern, wie z. B. Hypochlorit, wirksam sind und ohne wesentliche Einbusse der Effekte in Waschbädern mit nichtionogenen Waschmitteln, z. B. Alkylphenolpolyglykoläthern, verwendet werden können.

Die erfindungsgemässen Verbindungen werden in Mengen von 0,005 bis 1 % oder mehr, bezogen auf das Gewicht des flüssigen oder pulver-, förmigen, fertigen Waschmittels, zugesetzt. Waschflotten, die die angegebenen Mengen der beanspruchten Aufheller enthalten, verleihen beim Waschen von Textilien aus Cellulosefasern, Polyamidfasern, hochveredelten Cellulosefasern, Polyesterfasern, Wolle etc. diesen einen brillanten Aspekt am Tageslicht.

Die Waschbehandlung wird beispielsweise wie folgt durchgeführt:

Die angegebenen Textilien werden während 1 bis 30 Minuten bei 20 bis 100 °C in einem Waschbad behandelt, das 1 bis 10 g/kg eines aufgebauten, zusammengesetzten Waschmittels und 0,05 bis 1 % bezogen auf das Waschmittelgewicht, der beanspruchten Aufhellmittel enthält. Das Flottenverhältnis kann 1 : 3 bis 1 : 50 betragen. Nach dem Waschen wird wie üblich gespült und getrocknet. Das Waschbad kann als Bleichzusatz 0,2 g/I Aktivchlor (z. B. als Hypochlorit) oder 0,1 bis 2 g/l Natriumperborat enthalten.

Die nachfolgenden Beispiele erläutern die Herstellung der erfindungsgemässen Verbindungen sowie deren Verwendung weiter. In diesen Beispielen, ebenso wie in der übrigen Beschreibung, sind Prozent-und Teilangaben immer auf das Gewicht bezogen, sofern nichts anderes vermerkt ist. Schmelz- und Siedepunkte sind, wenn nicht anders angegeben, unkorrigiert.

### Beispiel 1

Zu einer Mischung von 6,2 g 4-(2-Cyanostyryl)-biphenyl-4'-aldehyd und 7,4 der Verbindung der Formel 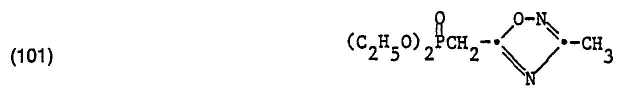 (Gehalt: 70,1 %) in 80 ml Dimethylformamid tropft man nach Verdrängen der Luft durch Stickstoff unter Rühren 5,4 g einer methanolischen 30 %-igen Natriummethylat-lösung, so dass die Temperatur nicht über 40 °C ansteigt. Man hält die Temperatur 2 Stunden bei 40-45 °C, kühlt mit Eiswasser ab und versetzt die Reaktionsmischung mit 80 ml Wasser. Das ausgefallene Produkt wird abgenutscht, wiederholt mit Methanol und Wasser gewaschen und im Vakuum bei 100 °C getrocknet. Man erhält 7,0 der Verbindung der Formel 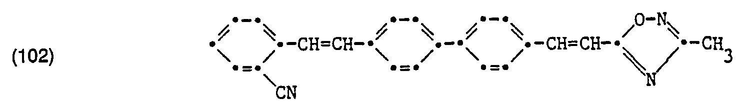 in Form hellgelber Kristalle mit einem Schmelzpunkt von 178-180 °C (nach einer Umkristallisation aus Xylol).

In analoger Weise erhält man aus den entsprechend substituierten 4-Styrylbiphenyl-4'-aldehyden und den Heterocyclylmethylphosphonaten vom Typ der Formel (101) die in Tabelle 1 aufgeführten Verbindungen der Formel

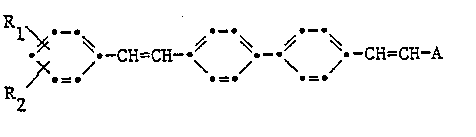

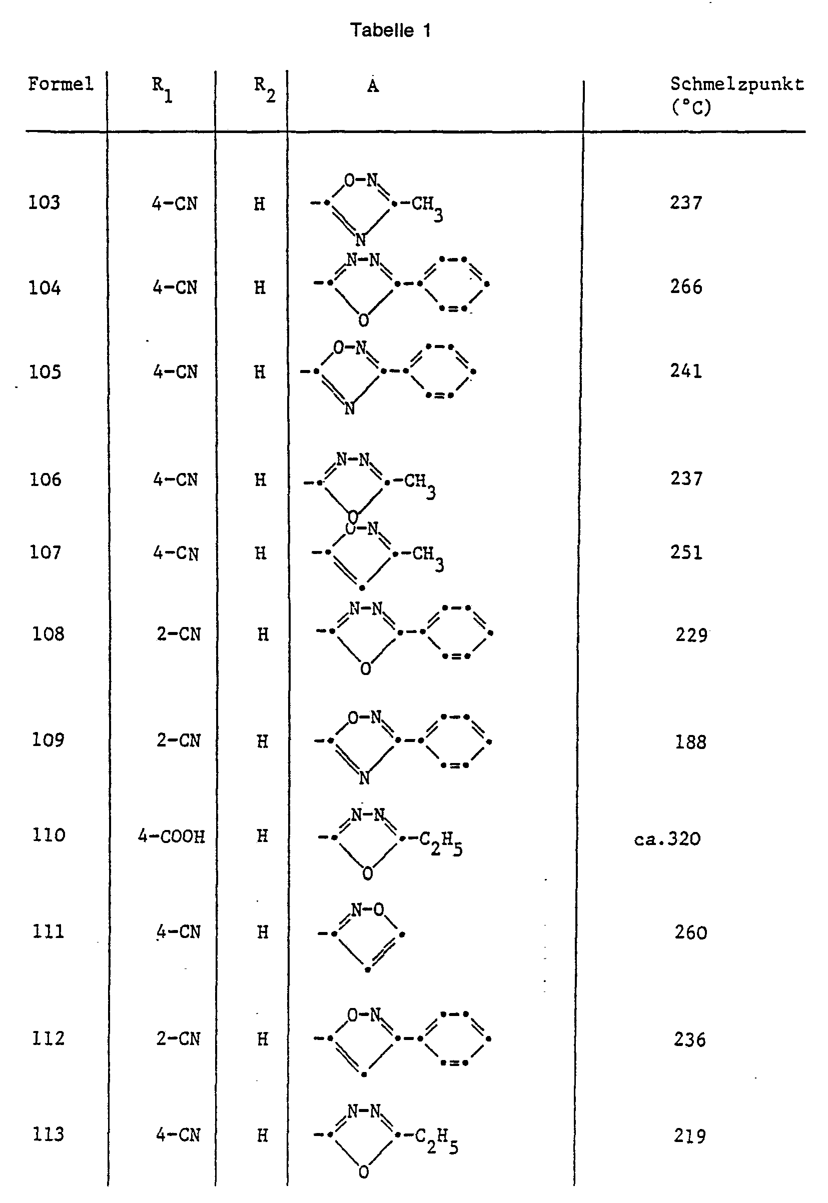

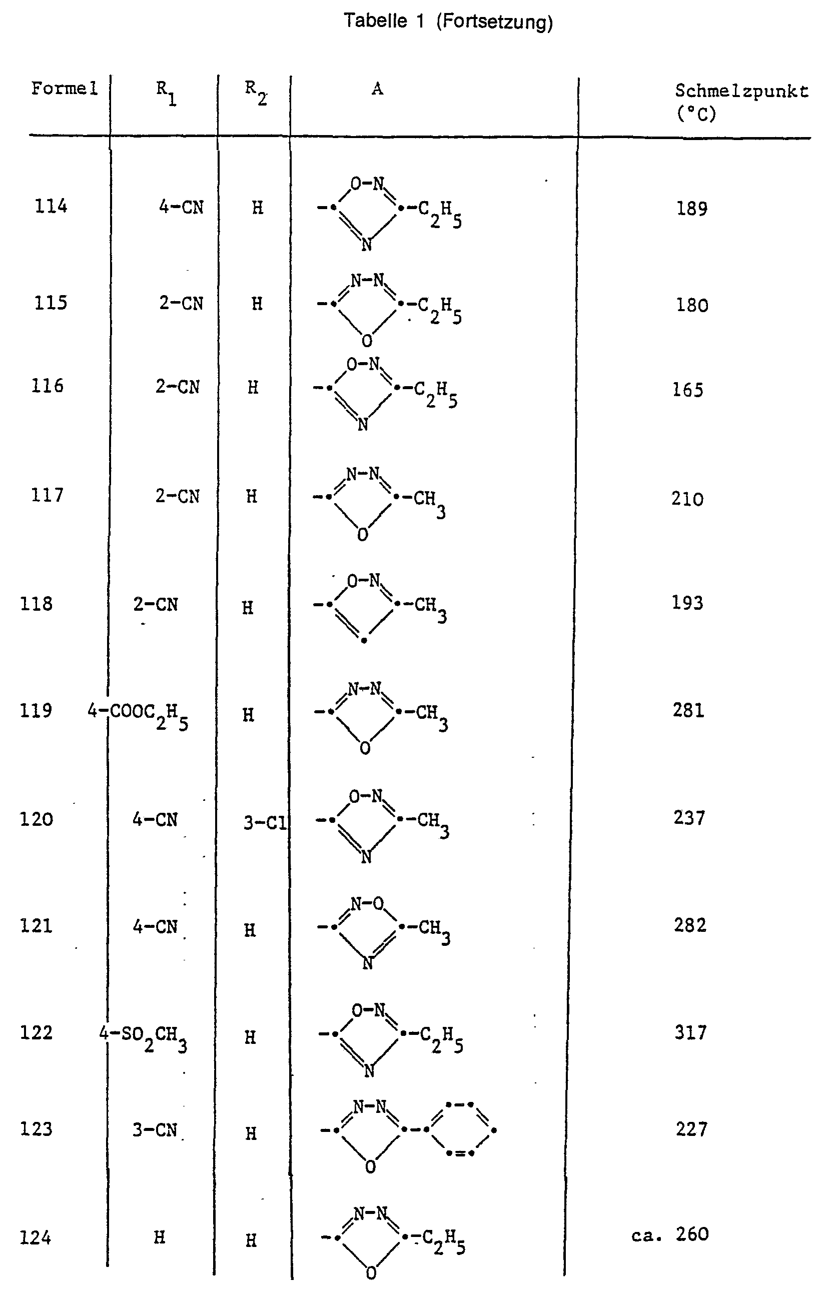

Den zur Herstellung der Verbindung der Formel (122) benötigten 4-(4-Methylsulfonylstyryl)-biphenyl-4'-aldehyd der Formel 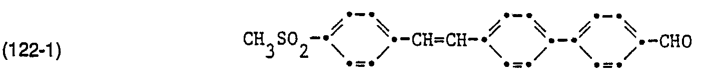 erhält man wie folgt : Zu einer Suspension von 21,0 g Biphenyl-4,4'-dialdehyd in 200 ml Methanol fügt man bei Raumtemperatur unter Rühren und Ueberleiten von Stickstoff 34,7 g einer 30 %-igen methanolischen Natriummethylatlösung und dann im Verlauf einer Stunde eine Lösung von 35,2 g des Phosphonates der Formel 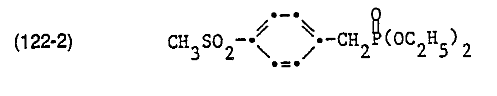 (Gehalt: 87 %) in 50 ml Methanol. Nach 20-stündigem Nachrühren bei Raumtemperatur wird das Reaktionsprodukt abgenutscht, wiederholt mit Methanol gewaschen und getrocknet. Zur Reinigung wird es mit heissem Methylenchlorid im Soxhletapparat über Nacht extrahiert, der eingedampfte Extrakt in Methanol verrührt und abgenutscht. Nach dem Trocknen im Vakuum bei 100 °C erhält man 26,3 eines blassgelben Produktes. Es lässt sich aus Aethylenglykolmonomethyläther umkristallisieren und weist einen Schmelzpunkt von 243 °C auf.

Nach diesem Verfahren lassen sich die meisten übrigen Ausgangsverbindungen der Formel (3) (Z, = CHO) herstellen, sofern sie nicht ohnehin aus der Literatur bekannt sind.

Den zur Herstellung der Verbindung der Formel (110) benötigten Aldehyd der Formel 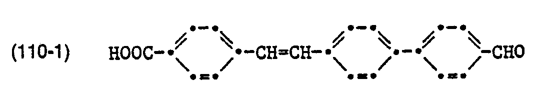 (Schmelzpunkt ca. 310 °C) erhält man z. B. durch Verseifen des entsprechenden Aethylesters in einer Mischung von Dioxan und konz. Salzsäure im Volumenverhältnis 6 : 1 bei Rückflusstemperatur.

Den zur Herstellung der Verbindung der Formel (125) benötigten 4'-Styryl-biphenyl-4-aldehyd erhält man z. B. durch Umsetzung von 4,4'-Biphenyl-dialdehyd mit Benzyl-triphenyl-phosphoniumchlorid in Gegenwart von Natriummethylat in Methanol nach Wittig.

### Beispiel 2

Zu einer Lösung von 9,3 g 4-(4-Cyanostyryl)-biphenyl-4'-aldehyd und 3,2 g 2-Methylpyrimidin (Gehalt: 94 %) in 100 ml Dimethylformamid fügt man unter Rühren und Ueberleiten von Stickstoff 6,7 g Kalium- tert.-butylat und lässt die Temperatur auf 50 °C ansteigen. Nach 2-stündigem Rühren bei 50 °C wird die Mischung im Eisbad abgekühlt und mit 70 ml Wasser versetzt. Das ausgefallene Produkt wird abgesaugt, wiederholt mit Methanol und Wasser gewaschen, im Vakuum bei 100 °C getrocknet und aus o-Dichlorbenzol umkristallisiert. Man erhält 4,9 g eines hellgelben, kristallinen Produktes der Formel 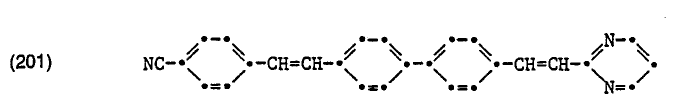 mit einem Schmelzpunkt von 309 °C.

Verwendet man in der vorstehenden Herstellungsvorschrift an Stelle des angegebenen Aldehyds dieselbe Menge 4-(2-Cyanostyryl)-biphenyl-4'-aldehyd und verfährt sonst wie beschrieben, so erhält man nach Umkristallisation aus Aethylenglykolmonomethyläther und Xylol die Verbindung der Formel 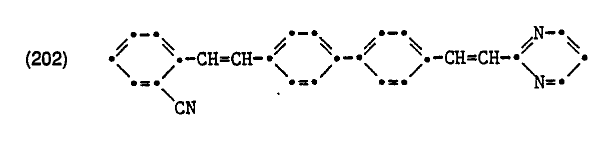 mit einem Schmelzpunkt von 220 °C.

In analoger Weise erhält man unter Verwendung der entsprechend substituierten Ausgangsprodukte die in Tabelle 2 aufgeführten Verbindungen der Formel

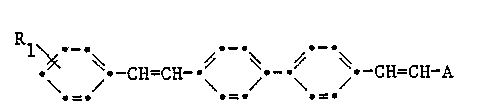

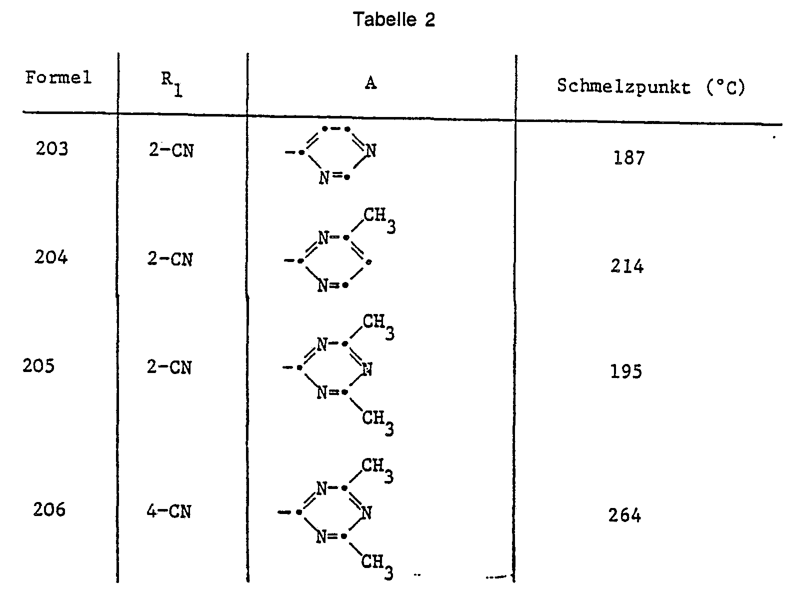

### Beispiel 3

Verfährt man nach den in den Beispielen 1 und 2 angegebenen Herstellungsvorschriften unter Verwendung der entsprechend substituierten Ausgangsprodukte, so erhält man die in Tabelle 3 aufgeführten Verbindungen der Formel

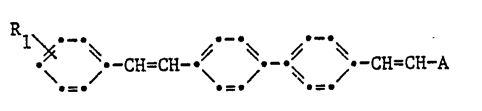

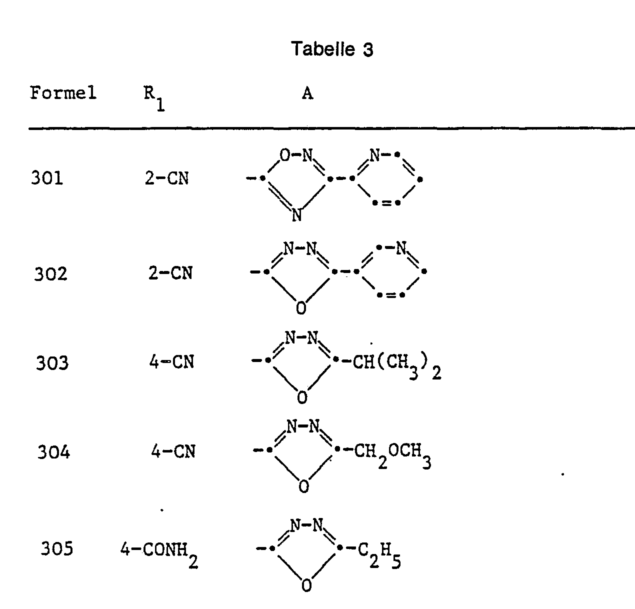

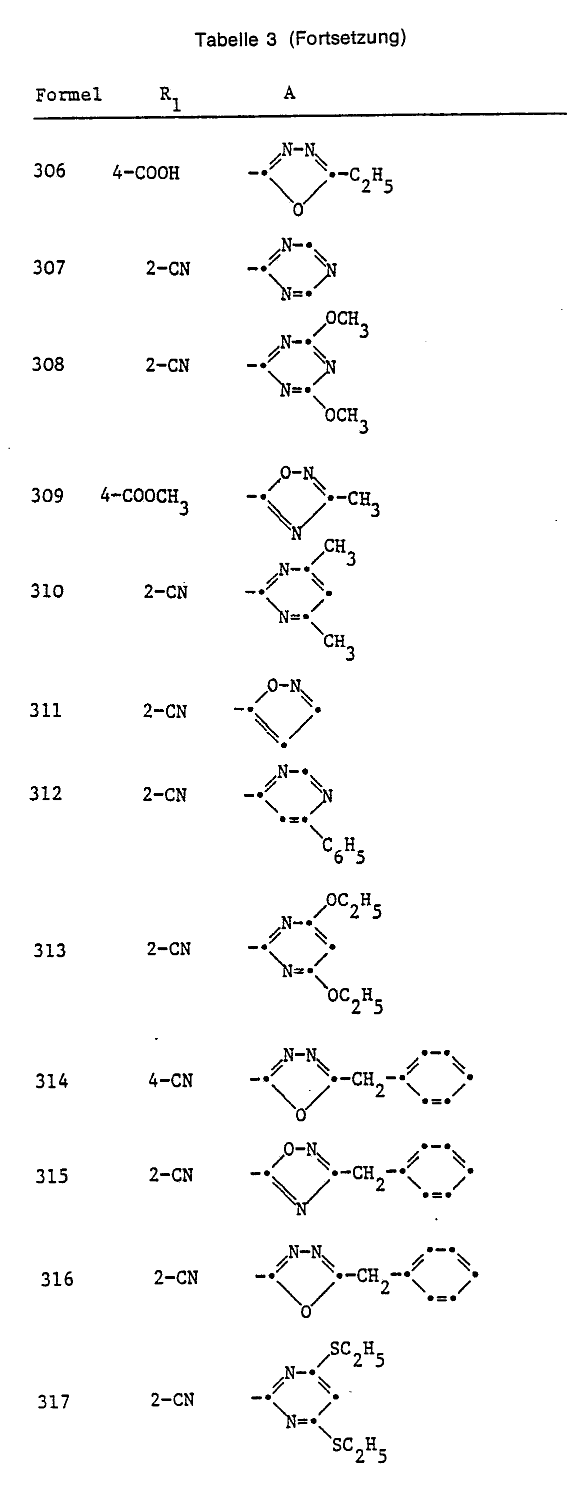

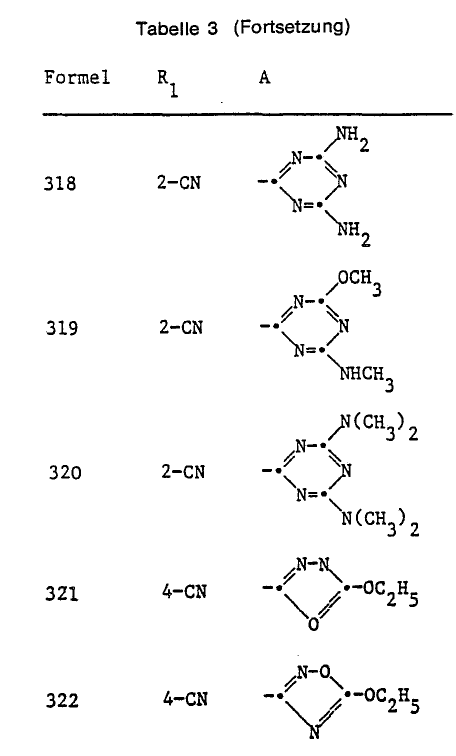

### Beispiel 4

Polyestergewebe wird auf einem Färbeapparat bei einem Flottenverhältnis von 1 : 20 mit einem wässrigen Bad behandelt, welches 0,1 %, bezogen auf das Gewebegewicht, der Verbindung der Formel (103) und 1 g/Liter des Kondensationsproduktes von 35 Mol Aethylenoxid mit 1 Mol Stearylalkohol enthält. Man erwärmt nun das Bad innerhalb 30 Minuten von 40 auf 130 °C, behält es während 30 Minuten auf dieser Temperatur und kühlt es dann innerhalb 15 Minuten auf 15 °C ab. Das Gewebe wird anschliessend in fliessendem deionisiertem Wasser gespült und bei 70 °C getrocknet. Das so behandelte Polyestergewebe weist einen hohen Aufhelleffekt auf.

Verwendet man in dieser Vorschrift an Stelle der genannten Verbindung der Formel (103) eine solche der Formel (102), (106), (116), (117), (119), (122) oder (202), so erhält man ähnlich gute Aufhelleffekte.

### Beispiel 5

Polyestergewebe wird bei Raumtemperatur mit einer wässerigen Dispersion foulardiert, die im Liter 0,5g der Verbindung der Formel (103) sowie 1 g eines Anlagerungsproduktes aus etwa 8 Mol Aethylenoxid an 1 Mol p-tert.-Octylphenol enthält. Die Flüssigkeitsaufnahme beträgt 60 bis 70%. Das Gewebe wird bei 80 °C getrocknet und anschliessend 30 Sekunden lang auf 220 °C erhitzt. Das so behandelte Gewebe weist einen ausgezeichneten Aufhelleffekt auf. Verwendet man in dieser Vorschrift an Stelle der genannten Verbindung der Formel (103) eine solche der Formel. (102), (106), (112), (116), (117), (119), (120), (121) oder (201), so erhält man ähnlich gute Aufhelleffekte.

### Beispiel 6

Polyestergewebe wird bei Raumtemperatur mit einer wässrigen Dispersion foulardiert, die im Liter 0,5 g einer Aufhellermischung, bestehend aus 2 Teilen der Verbindung der Formel (103) und 1 Teil der Verbindung der Formel 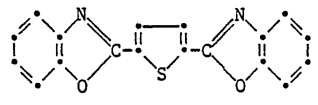 sowie 1 g eines Anlagerungsproduktes aus etwa 8 Mol Aethylenoxid an 1 Mol p-tert.-Octylphenol enthält. Die Flüssigkeitsaufnahme beträgt 60-70 %. Das Gewebe wird bei 100 °C getrocknet und anschliessend 30 Sekunden lang auf 200 °C erhitzt.

Das so behandelte Gewebe weist einen ausgezeichneten Aufhelleffekt von guter Lichtechtheit auf.

### Beispiel 7

Man behandelt Polyestergewebe nach der in Beispiel 6 angegebenen Vorschrift, ersetzt jedoch die dort verwendete Aufhellermischung durch eine der in der nachfolgenden Tabelle 4 angeführten Aufhellermischungen A-K.

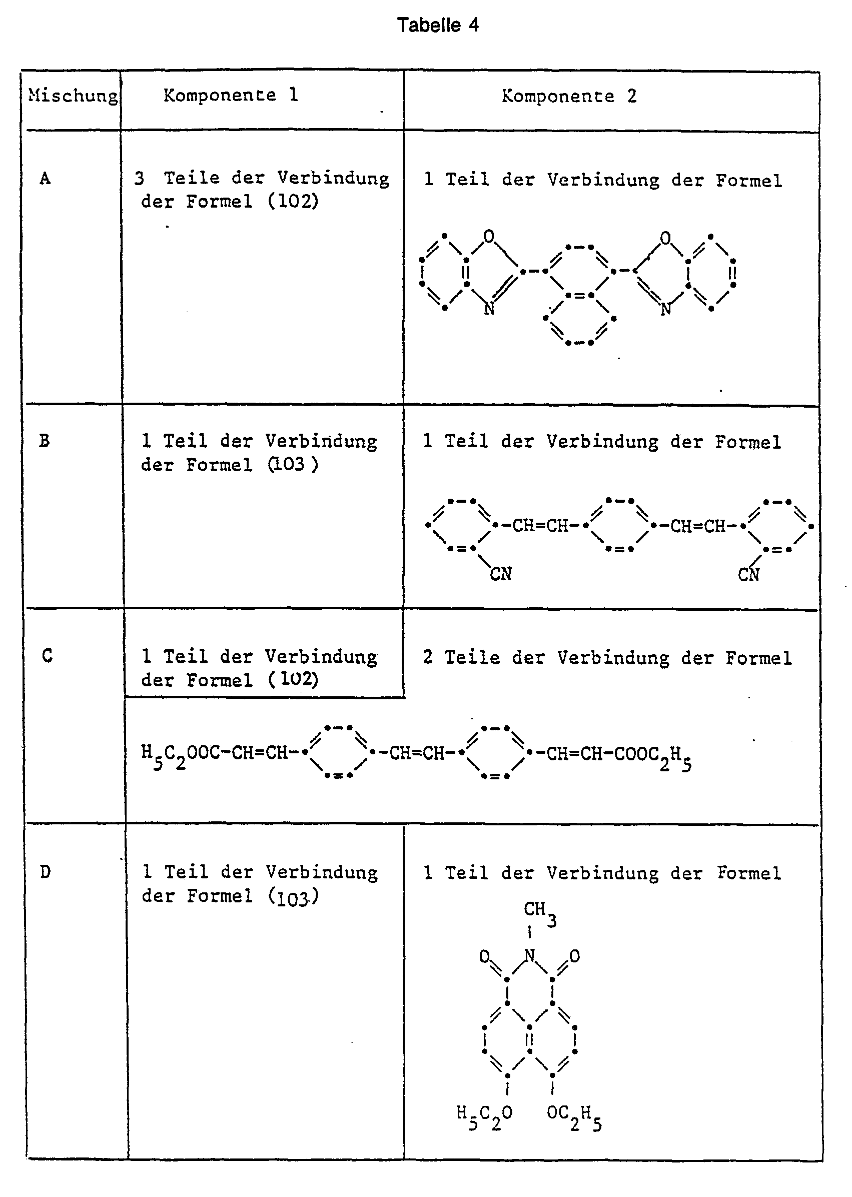

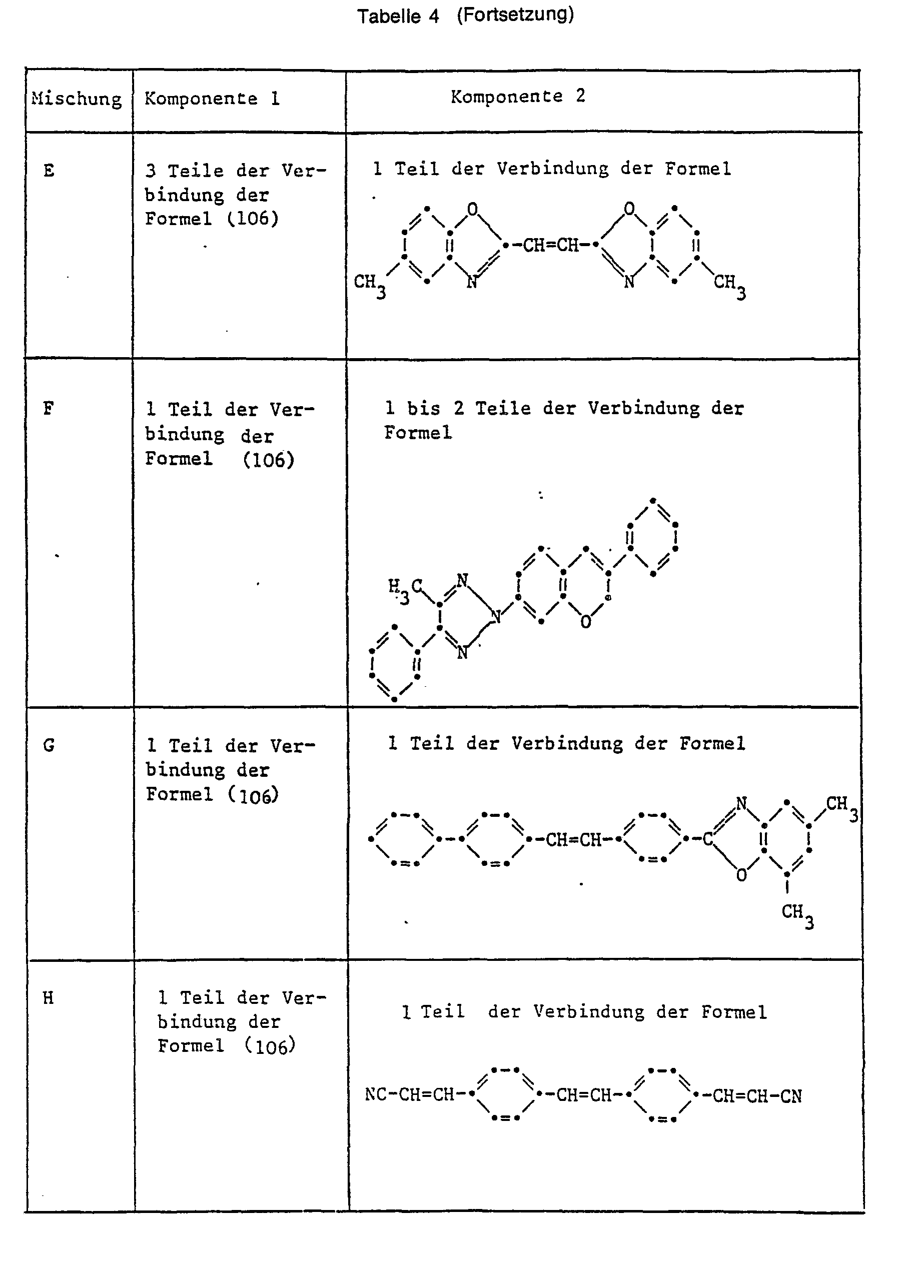

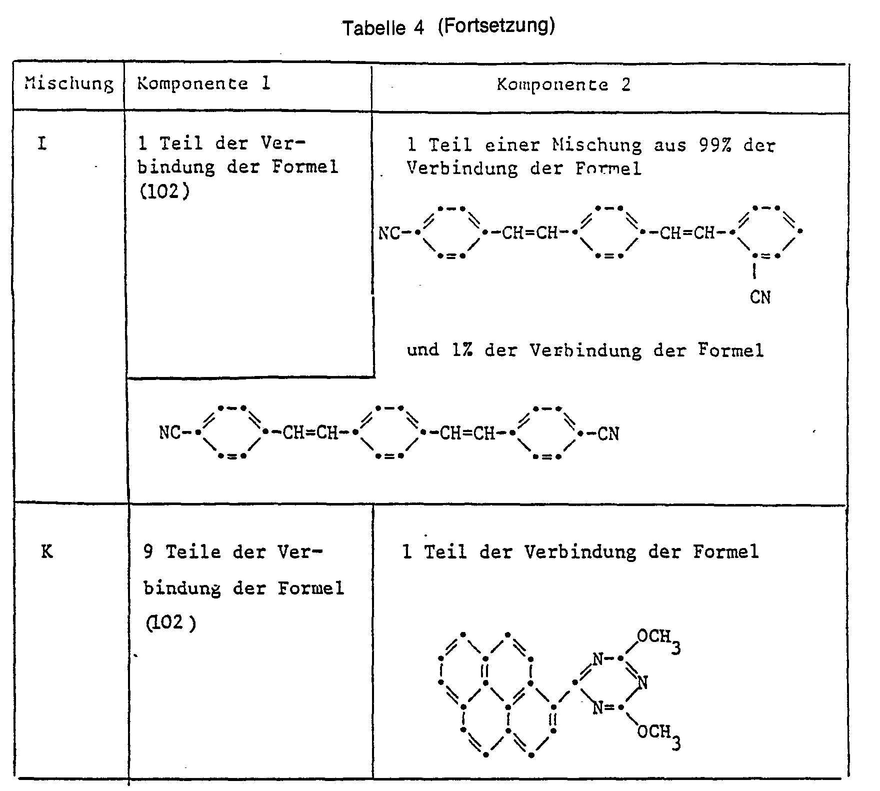

Das mit der jeweiligen Aufheller-Mischung behandelte Polyestergewebe weist jeweils einen ausgezeichneten Aufhelleffekt auf.

### Beispiel 8

Polyamid 6,6-Webtricot wird auf einem Färbeapparat bei einem Flottenverhältnis von 1 : 20 mit einem wässrigen Bad behandelt, welches 0,2 %, bezogen auf das Gewicht des Gewebes, einer Verbindung der Formel (106), (113), (117) oder (118) und 1 g/I eines Alkylphenolpolyglykoläthers enthält. Man erwärmt das Bad innerhalb von 30 Minuten auf 130 °C, behält es während 30 Minuten bei dieser Temperatur und kühlt dann innerhalb von 15 Minuten auf 40 °C ab. Das Gewebe wird anschliessend in fliessendem deionisiertem Wasser gespült und bei 180 °C mit dem Bügeleisen getrocknet. Das so behandelte Polyamidgewebe weist in allen 4 Fällen einen hohen Aufhelleffekt auf.

## Patentansprüche

1. 4-Heterocyclylvinyl-4'-styryl-biphenyle der Formel 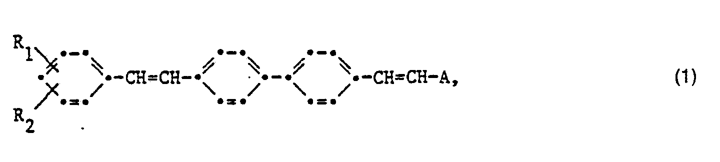 worin A einen unsubstituierten oder nicht-chromophor substituierten Isoxazolyl-, Oxadiazolyl-, Pyrimidinyl- oder Triazinylrest, R₁ Wasserstoff oder einen nicht-chromophoren Substituenten und R₂ Wasserstoff, Halogen oder Alkyl bedeuten.

2. 4-Heterocyclylvinyl-4'-styryl-biphenyle nach Anspruch 1, worin R, Wasserstoff oder einen nicht-chromophoren Substituenten 2. Ordnung, R₂ Wasserstoff, Chlor oder Alkyl und A einen Rest der Formel 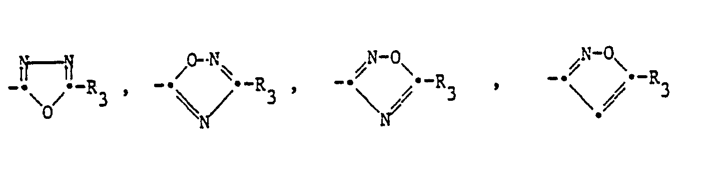 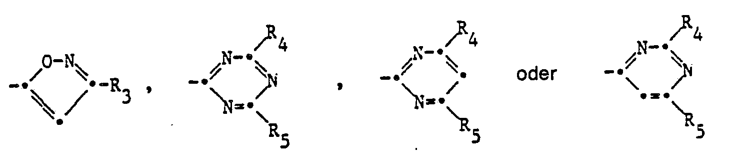 bedeuten, worin R₃, R₄ und R₅ unabhängig voneinander für Wasserstoff oder einen nicht-chromophoren Substituenten stehen.

3. 4-Heterocyclylvinyl-4'-styryl-biphenyle nach Anspruch 1 oder 2, worin R₁ Wasserstoff, Alkylsulfonyl, Phenylsulfonyl, Alkoxysulfonyl, Cyano, Trifluormethyl, die Sulfogruppe und deren Salze, die Carboxylgruppe und deren Salze oder eine Gruppe der Formel ―COOY₁, ―CONY₁Y₂ oder ―SO₂NY₁Y₂, worin Y₁ und Y₂ unabhängig voneinander Wasserstoff, Alkenyl, Cycloalkyl, unsubstituiertes oder nicht-chromophor substituiertes Alkyl, Phenylalkyl, oder Y₁ und Y₂ zusammen mit dem Stickstoffatom, an das sie gebunden sind, für einen 5- oder 6-gliedrigen gesättigten heterocyclischen Ring stehen, der gegebenenfalls noch zusätzlich 1 oder 2 Heteroatome als Ringglieder enthalten und gegebenenfalls durch Alkylgruppen substituiert sein kann, R₂ Wasserstoff, Chlor oder Alkyl, R₃ Wasserstoff, Alkyl, Cycloalkyl, Phenyl, Benzyl, Pyridyl oder Alkoxyalkyl und R₄ und R₅ unabhängig voneinander Wasserstoff, Alkyl, Phenyl, Alkoxy, Halogen, Amino, Mono- oder Dialkylamino, Alkylmercapto, Hydroxy oder Alkoxyalkyl bedeuten, vorzugsweise solche, worin R₁ Wasserstoff, C₁-C₄-Alkylsulfonyl, Phenylsulfonyl, C₁-C₄-Alkoxysulfonyl, Cyano, die Sulfogruppe und deren Salze, die Carboxylgruppe und deren Salze oder eine Gruppe der Formel ―COOY'₁, -CONY'1Y'2 oder ―SO₂NY'₁Y'₂, worin Y'₁ und Y'₂ unabhängig voneinander für Wasserstoff, C₁-C₄-Alkyl oder Benzyl stehen, R₂ Wasserstoff, Chlor oder C₁-C₄-Alkyl, R₃ Wasserstoff, C₁-C₄-Alkyl, Benzyl, Phenyl, Pyridyl oder C₂-C₈-Alkoxyalkyl und R₄ und R₅ unabhängig voneinander Wasserstoff, C₁-C₄-Alkyl, Phenyl, C₁-C₄-Alkoxy, Chlor, C₂-C₈-Alkoxyalkyl, C₁-C₄-Alkylmercapto, Amino, C₁-C₄-Alkylamino oder C₂-C₆-Dialkylamino bedeuten.

4. 4-Heterocyclylvinyl-4'-styryl-biphenyle nach Anspruch 3, worin R₁ Wasserstoff, C₁-C₄-Alkylsulfonyl, Cyano, Carboxy, C₂-C₅-Alkoxycarbonyl oder Aminocarbonyl, R₂ Wasserstoff oder Chlor, R₃ Wasserstoff, C₁-C₄-Alkyl, Benzyl, Phenyl, Pyridyl oder C₂-C₆-Alkoxyalkyl und R₄ und R₅ unabhängig voneinander Wasserstoff, C₁-C₄-Alkyl, Phenyl, C₁-C₄-Alkoxy, C₁-C₄-Alkylmercapto, Amino, C₁-C₄-Alkyl- amino oder C₂-C₆-Dialkylamino bedeuten.

5. 4-Heterocyclylvinyl-4'-styryl-biphenyle nach einem der Ansprüche 1-4, worin A einen unsubstituierten oder substituierten 1,3,4-Oxadiazol-2-yl-, 1,2,4-Oxadiazol-5-yl-, 1,2,4-Oxadiazol-3-yl-, 1,2-Oxazol-3-yl-, 1,2-Oxazol-5-yl- oder 1,3-Pyrimidin-2-ylrest, vorzugsweise einen 1,3,4-Oxadiazol-2-yl-, 1,2,4-Oxadiazoyl-5-yl- oder 1,2,4-Oxadiazol-3-ylrest bedeutet.

6. 4-Heterocyclylvinyl-4'-styryl-biphenyle nach Anspruch 5 der Formel 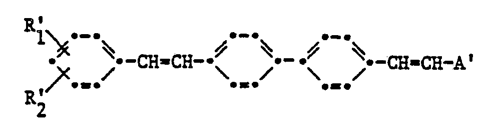 worin R'₁, C₁-C₄-Alkylsulfonyl, Cyano, Carboxy oder C₂-C₅-Alkoxycarbonyl, R'₂ Wasserstoff oder Chlor und A' einen Rest der Formel 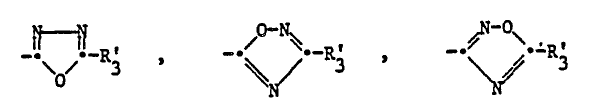 bedeuten, worin R'₃ für Wasserstoff, C₁-C₄-Alkyl oder Phenyl, vorzugsweise, für C₁-C₄-Alkyl steht.

7. 4-Heterocyclylvinyl-4'-styryl-biphenyle nach einem der Ansprüche 1-6, worin R₁ bzw. R'₁ für Cyano, C₁-C₄-Alkylsulfonyl oder C₂-C₅-Alkoxycarbonyl, vorzugsweise für Cyano, und R₂ bzw. R'₂ für Wasserstoff steht.

8. Verfahren zur Herstellung der 4-Heterocyclylvinyl-4'-styryl-biphenyle der in Anspruch 1 definierten Formel (1), dadurch gekennzeichnet, dass man eine Verbindung der Formel 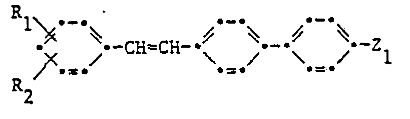 mit einer Verbindung der Formel Z₂-A, oder eine Verbindung der Formel 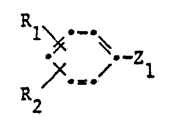 mit einer Verbindung der Formel 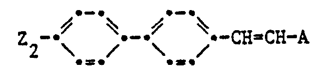 umsetzt, wobei in obigen Formeln R_{1'} R₂ und A wie in Anspruch 1 definiert sind und jeweils einer der beiden Substituenten Z, und Z₂ die Gruppe 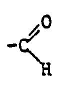 und der andere Methyl, Carboxymethyl (oder ein funktionelles Derivat davon) oder eine Gruppe der Formel 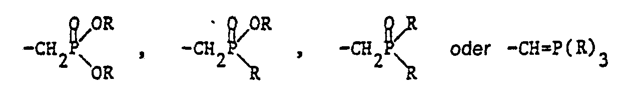 bedeutet, worin R unsubstituiertes oder substituiertes C₁-C₅-Alkyl oder unsubstituiertes oder substituiertes Phenyl bedeutet.

9. Verfahren zur Herstellung von 4-Heterocyclylvinyl-4'-styryl-biphenylen der in Anspruch 1 definierten Formel (1), worin A eine Gruppe der Formel 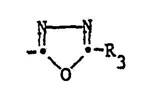 bedeutet, wobei R₃ für Wasserstoff oder einen nicht-chromophoren Substituenten steht, dadurch gekennzeichnet, dass man eine Carbonsäure der Formel 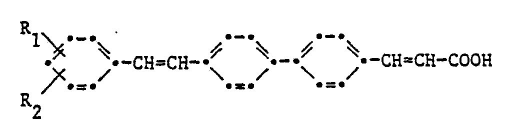 oder ein funktionelles Derivat dieser Säure mit einem Carbonsäurehydrazid der Formel H₂N―NH―CO―R₃, oder eine Verbindung der Formel 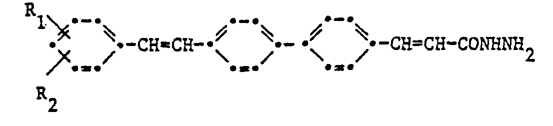 mit einer Carbonsäure der Formel HOOC-R₃ oder einem funktionellen Derivat dieser Säure umsetzt, wobei R₁ und R₂ wie in Anspruch 1 und R₃ wie oben definiert sind, und das erhaltene Kondensationsprodukt in Gegenwart eines wasserabspaltenden Mittels cyclisiert.

10. Verfahren zur Herstellung von 4-Heterocyclylvinyl-4'-styryl-biphenylen der in Anspruch 1 definierten Formel (1), worin A eine Gruppe der Formel 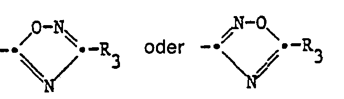 bedeutet, wobei R₃ für Wasserstoff oder einen nicht-chromophoren Substituenten steht, dadurch gekennzeichnet, dass man eine Carbonsäure der Formel 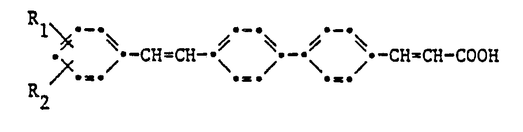 oder ein funktionelles Derivat dieser Säure mit einem Amidoxin der Formel 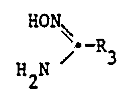 oder eine Verbindung der Formel 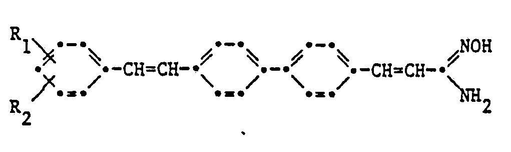 mit einer Carbonsäure der Formel HOOC-R₃ oder einem funktionellen Derivat dieser Säure umsetzt, wobei R₁ und R2 wie in Anspruch 1 und R₃ wie oben definiert ist, und das erhaltene Kondensationsprodukt in Gegenwart eines wasserabspaltenden Mittels cyclisiert.

11. Mittel zum optischen Aufhellen von hochmolekularen organischen Materialien, das ein oder mehrere in den Ansprüchen 1-7 definierte(s) 4-Heterocyclylvinyl-4'-styryl-biphenyl(e) enthält.

12. Mittel nach Anspruch 11, enthaltend oder bestehend aus ein(em) oder mehrere(n) in den Ansprüchen 1-7 definierte(n) 4-Heterocyclylvinyl-4'-styryl-biphenyle(n) und einen (einem) oder mehrere(n) optische(n) Aufheller(n) aus der Klasse der 1,4-Bis-styrylbenzole, 4-Benzoxazolylstilbene, 4,4'-Divinylstilbene, Naphthalimide, 4,4'-Bis-styrylbiphenyle, 4,4'-Bis-triazolyl-stilbene, Bis-benzoxazolylthiophene, - naphthaline und -äthylene, Oxadiazolyl-stilbene, Naphthotriazol-2-yl-stilbene, Triazinyl-pyrene, 2-Styrylbenzoxazole und der Cumarine, insbesondere der Triazolyl- oder Pyrazolyl-cumarine, wobei die Aufheller aus den genannten Klassen vorzugsweise Polyesteraufheller sind.

13. Mittel nach Anspruch 12, enthaltend, als Aufheller-Aktivsubstanz, 10-99 %, insbesondere 30-70%, eines in den Ansprüchen 1-7 definierten 4-Heterocyclylvinyl-4'-styryl-biphenyls und 90-1 %, insbesondere 70-30 %, eines oder mehrerer optischer Aufheller aus den in Anspruch 12 definierten Klassen.

14. Mittel nach Anspruch 12, das als Polyesteraufheller 1,4-Bis-(benzoxazol-2-yl)-naphthalin, 1-(4'-Methoxycarbonylphenyl)-2-(5',6'-dimethylbenzoxazol.2'-yl) äthylen, 4,4'-Bis-(äthoxycarbonyl-vinyl)-stilben, 4,4'-Bis-(cyanovinyl)-stilben, 1,4-Bis-(2'-cyanostyryl)-benzol, 1,5-Bis-(benzoxazol-2'-yl)-thiophen, 4-Phenyl-4'-(5",7"-dimethylbenzoxazol-2"-yl)-stilben, 1,2-Bis-(5'-methyl-benzoxazol-2'-yl)-äthylen, 4-(Benzoxazol-2'-yl)-4'-(3"-methyl-1",2",4"-oxadiazol-5"-yl) stilben oder 2,4-Dimethoxytriazin-6-yl-pyren enthält, wobei das Mengenverhältnis zum jeweiligen 4-Heterocyclylvinyl-4'-styrylbiphenyl vorzugsweise 1 : 2 bis 2 : 1 beträgt.

15. Verfahren zum optischen Aufhellen von natürlichen, halbsynthetischen oder synthetischen hochmolekularen organischen Substraten, insbesondere Textilfasern, dadurch gekennzeichnet, dass man eine in den Ansprüchen 1-7 definierte Verbindung oder eine Aufhellermischung nach einem der Ansprüche 12-14 den Substraten einverleibt oder auf die Substrate, vorzugsweise Textilfasern, aufbringt, wobei die aufzuhellenden Substrate insbesondere aus Polyester oder Polyamid bestehen.

16. Verfahren nach Anspruch 15, dadurch gekennzeichnet, dass man dem aufzuhellenden Substrat 0,001 bis 2, vorzugsweise 0,01 bis 0,5 % des Aufhellers bzw. der Aufhellermischung, bezogen auf das Gewicht des Substrats, einverleibt oder auf das Substrat aufbringt.

17. Verwendung von in den Ansprüchen 1 bis 7 definierten Verbindungen zum optischen Aufhellen von natürlichen, halbsynthetischen oder synthetischen hochmolekularen organischen Materialien, insbesondere aus Polyester oder Polyamid, vor allem aus Polyesterfasern.

## Claims

1. A 4-heterocyclylvinyl-4'-styryl-biphenyl of the formula 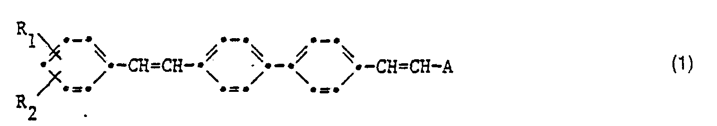 in which A is an isoxazolyl, oxadiazolyl, pyrimidinyl or triazinyl radical which is unsubstituted or substituted by a non-chromophoric substituent, R₁ is hydrogen or a non-chromophoric substituent and R₂ is hydrogen, halogen or alkyl.

2. A 4-heterocyclylvinyl-4'-styryl-biphenyl according to Claim 1, in which R, is hydrogen or a second- order non-chromophoric substituent, R₂ is hydrogen, chlorine or alkyl and A is a radical of the formula 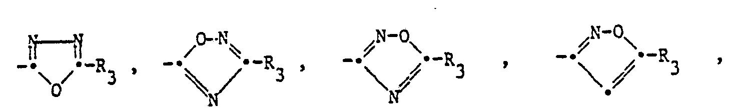 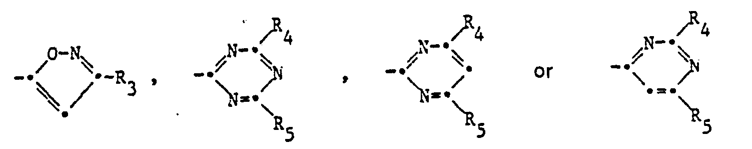 in which R₃, R₄ and Rₛ independently of one another are hydrogen or a non-chromophoric substituent.

3. A 4-heterocyclylvinyl-4'-styryl-biphenyl according to Claim 1 or 2, in which R₁ is hydrogen, alkylsulfonyl, phenylsulfonyl, alkoxysulfonyl, cyano, trifluoromethyl, a sulfo group and salts thereof, a carboxyl group and salts thereof or a group of the formula ―COOY₁, ―CONY₁Y₂ or ―SO₂NY₁Y₂ in which Y₁ and Y₂ independently of one another are hydrogen, alkenyl, cycloalkyl, alkyl which is unsubstituted or substituted by a non-chromophoric substituent, or phenylalkyl, or Y₁ and Y₂, together with the nitrogen atom to which they are linked, are a 5-membered or 6-membered saturated heterocyclic ring which, if appropriate, can also contain 1 or 2 hetero-atoms as additional ring members and can be unsubstituted or substituted by alkyl groups, R₂ is hydrogen, chlorine or alkyl, R₃ is hydrogen, alkyl, cycloalkyl, phenyl, benzyl, pyridyl or alkoxyalkyl, and R₄ and Rₛ independently of one another are hydrogen, alkyl, phenyl, alkoxy, halogen, amino, mono- or dialkylamino, alkylmercapto, hydroxyl or alkoxyalkyl, preferably those in which R₁ is hydrogen, C₁-C₄alkylsulfonyl, phenylsulfonyl, C₁-C₄alkoxysulfonyl, cyano, a sulfo group and salts thereof, a carboxyl group and salts thereof or a group of the formula ―COOY'₁, ―CONY'₁Y'₂ or ―SO₂NY'₁Y'₂, in which Y'₁ and Y'₂ independently of one another are hydrogen, C₁-C₄alkyl or benzyl, R₂ is hydrogen, chlorine or C₁-C₄-alkyl, R₃ is hydrogen, C₁-C₄alkyl, benzyl, phenyl, pyridyl or C₂-Cₛ-alkoxyalkyl, and R₄ and Rₛ independently of one another are hydrogen, C₁-C₄alkyl, phenyl, C₁-C₄alkoxy, chlorine, C₂-C₈-alkoxyalkyl, C₁-C₄-alkymercapto, amino, C₁-C₄alkylamino or C₂-C₆dialkylamino.

4. A 4-heterocyclylvinyl-4'-styryl-biphenyl according to Claim 3, in which R, is hydrogen, C₁-C₄alkylsulfonyl, cyano, carboxyl, C₂-Cₛ-alkoxycarbonyl or aminocarbonyl, R₂ is hydrogen or chlorine, R₃ is hydrogen, C₁-C₄alkyl, benzyl, phenyl, pyridyl or C₂-C₆alkoxyalkyl, and R₄ and R₅ independently of one another are hydrogen, C₁-C₁alkyl, phenyl, C₁-C₄alkoxy, C₁-C₄alkylmercapto, amino, C₁-C₄alkylamino or C₂-C₆dialkylamino.

5. A 4-heterocyclylvinyl-4'-styryl-biphenyl according to any one of Claims 1-4, in which A is an unsubstituted or substituted 1,3,4-oxadiazol-2-yl, 1,2,4-oxadiazol-5-yl, 1,2,4-oxadiazol-3-yl, 1,2-oxazol-3-yl, 1,2-oxazol-5-yl, or 1,3-pyrimidin-2-yl radical, preferably a 1,3,4-oxadiazol-2-yl, 1,2,4-oxadiazol-5-yl or 1,2,4-oxadiazol-3-yl radical.

6. A 4-heterocyclylvinyl-4'-styryl-biphenyl according to Claim 5, of the formula 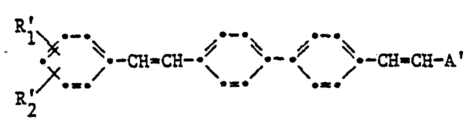 in which R'₁ is C₁-C₄alkylsulfonyl, cyano, carboxyl or C₂-C₅alkoxycarbonyl, R'₂ is hydrogen or chlorine and A is a radical of the formula 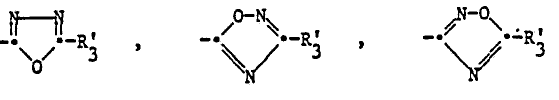 in which R'₃ is hydrogen, C₁-C₄alkyl or phenyl, preferably C₂-C₄alkyl.

7. A 4-heterocyclylvinyl-4'-styryl-biphenyl according to any one of Claims 1-6, in which R₁ or R'₁ is cyano, C₁-C₄alkylsulfonyl or C₂-C₅alkoxycarbonyl, preferably cyano and R₂ or R'₂ is hydrogen.

8. A process for preparing a 4-heterocyclyvinyl-4'-styryl-biphenyl of the formula (1) defined in Claim 1, which comprises reacting a compound of the formula 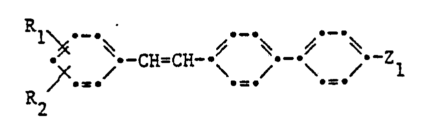 with a compound of the formula Zg-A, or reacting a compound of the formula 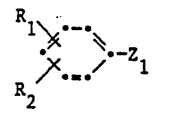 with a compound of the formula 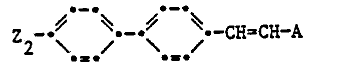 R₁, R₂ and A in the above formulae being as defined in Claim 1, and in each case one of the two substituents Z₁ and Z₂ being the group 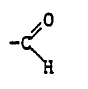 and the other being methyl, carboxymethyl (or a functional derivative thereof) or a group of the formula 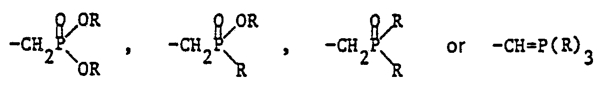 in which R is unsubstituted or substituted C₁-C₅alkyl or unsubstituted or substituted phenyl.

9. A process for preparing a 4-heterocyclylvinyl-4'-styryl-biphenyl of the formula (1) defined in Claim 1, in which A is a group of the formula 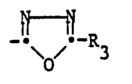 in which R₃ is hydrogen or a non-chromophoric substituent, which comprises reacting a carboxylic acid of the formula 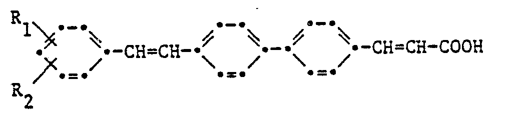 or a functional derivative of this acid with a carboxylic acid hydrazide of the formula H₂N-NH-CO-R₃, or reacting a compound of the formula 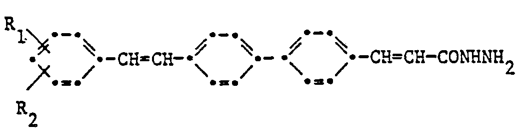 with a carboxylic acid of the formula HOOC-R₃ or a functional derivative of this acid, R₁ and R₂ being as defined in Claim 1 and R₃ being as defined above, and cyclising the resulting condensation product in the presence of a dehydrating agent.

10. A process for preparing a 4-heterocyclylvinyl-4'-styryl-biphenyl of the formula (1) defined in Claim 1, in which A is a group of the formula 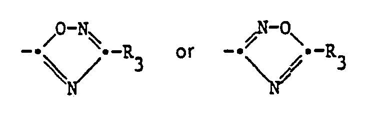 in which R₃ is hydrogen or a non-chromophoric substituent, which comprises reacting a carboxylic acid of the formula 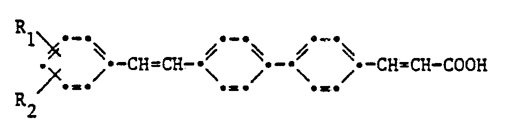 or a functional derivative of this acid with an amidoxime of the formula 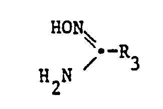 or reacting a compound of the formula 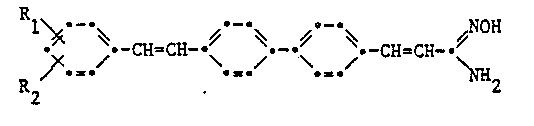 with a carboxylic acid of the formula HOOC-R₃ or a functional derivative of this acid, Rᵢ, and R₂ being as defined in Claim 1 and R₃ being as defined above, and cyclising the resulting condensation product in the presence of a dehydrating agent.

11. An agent for fluorescent brightening of high-molecular organic materials, which contains one or more 4-heterocyclylvinyl-4'-styryl-biphenyls as defined in any one of Claims 1-7.

12. An agent according to Claim 11, which contains or consists of one or more 4-heterocyclylvinyl-4'- styryl-biphenyl(s) as defined in any one of Claims 1-7 and one or more fluorescent brightener(s) from the classes of the 1,4-bis-styrylbenzenes, 4-benzoxazolylstilbenes, 4,4'-divinylstilbenes, naphthalimides, 4,4'- bis-styrylbiphenyls, .4,4'-bis-triazolyl-stilbenes, bis-benzoxazolyl-thiophenes, -naphthalenes and - ethylenes, oxadiazolyl-stilbenes, naphthotriazol-2-yl-stilbenes, triazinyl-pyrenes, 2-styryl-benzoxazoles and the coumarins, in particular the triazolyl- or pyrazolyl-coumarins, the brightener from the classes listed preferably being a polyester brightener.

13. An agent according to Claim 12, which contains, as the active brightener substance, 10-99 %, in particular 30-70 %, of a 4-heterocyclylvinyl-4'-styryl-biphenyl as defined in any one of Claims 1-7 and 90-1 %, in particular 70-30 %, of one or more optical brighteners from the classes defined in Claim 12.

14. An agent according to Claim 12, which contains, as the polyester brightener, 1,4-bis-(benzoxazol-2-yl)-naphthalene, 1-(4'-methoxycarbonylphenyl)-2-(5',6'-dimethylbenzoxazol-2'-yl)-ethylene, 4,4'-bis-(ethoxycarbonyl-vinyl)-stilbene, 4,4'-bis-(cyanovinyl)-stilbene, 1,4-bis-(2'-cyanostyryl)-benzene, 1,5-bis-(benzoxazol-2'-yl)-thiophene, 4-phenyl-4'-(5",7"-dimethylbenzoxazol-2''-yl)-stilbene, 1,2-bis-(5'-methylben- zoxazol-2'-yl)-ethylene, 4-(benzoxazol-2'-yl)-4'-(3"-methyl-1",2",4"-oxadiazol-5"-yl)-stilbene or 2,4-di- methoxytriazin-6-yl-pyrene, the quantity ratio to the particular 4-heterocyclylvinyl-4'-styryl-biphenyl being preferably 1 : 2 to 2 : 1.

15. A process for the fluorescent brightening of natural, semi-synthetic or synthetic high-molecular organic substrates, in particular textile fibres, which comprises incorporating a compound as defined in any one of Claims 1-7 or a brightener mixture according to any one of Claims 12-14 into the substrates or applying these to the substrates, preferably textile fibres, the substrates to be brightened consisting in particular of polyester or polyamide.

16. A process according to Claim 15, wherein 0.001 to 2 %, preferably 0.01 to 0.5 % of the brightener or brightener mixture, relative to the weight of the substrate, is incorporated in the substrate to be brightened or applied to the substrate.

17. The use of a compound as defined in any one of Claims 1 to 7 for the.fluorescent brightening of natural, semi-synthetic or synthetic high-molecular organic materials, in particular materials of polyester or polyamide, especially polyester fibres.

## Revendications

1. 4-hétérocyclyvinyl-4'-styryl-biphényles de formule 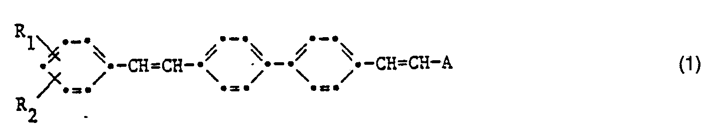 dans laquelle A représente un reste isoxazolyle, oxadiazolyle, pyrimidinyle ou triazinyle, non substitué ou à substituant(s) non chromophore(s), R₁ représente un hydrogène ou un substituant non chromophore et R₂ représente un hydrogène, un halogène ou un reste alkyle.

2. 4-hétérocyclylvinyl-4'-styryl-biphényles selon la revendication 1, où R₁ représente un hydrogène ou un substituant non chromophore d'ordre deux, R₂ représente un hydrogène, un chlore ou un reste alkyle et A représente un reste de formule : 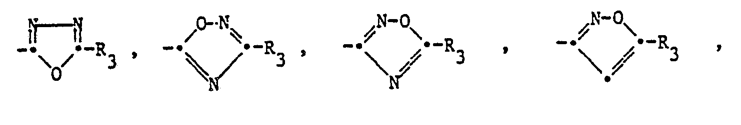 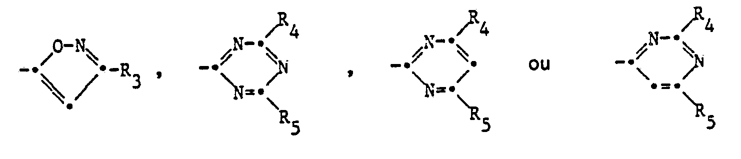 où R₃, R₄ et R₅ représentent, indépendamment l'un de l'autre, un hydrogène ou un substituant non chromophore.

3. 4-hétérocyclyvinyl-4'-styryl-biphényles selon les revendications 1 ou 2, où R₁ représente un hydrogène, un reste alkylsulfonyle, un reste phénylsulfonyle, un reste alcoxysulfonyle, le reste cyano, le reste trifluorométhyle, le groupe sulfo et ses sels, le groupe carboxyle et ses sels ou un groupe de formule ―COOY₁, ―CONY₁Y₂ ou ―SO₂NY₁Y₂, où Y₁ et Y₂ représentent, indépendamment l'un de l'autre, un hydrogène, un reste alcényle, un reste cycloalkyle, un reste alkyle non substitué ou à substituant non chromophore, un reste phénylalkyle, ou bien Y₁ et Y₂ représentent ensemble, avec l'atome d'azote auquel ils sont liés, un noyau hétérocyclique saturé de 5 ou 6 chaînons, qui peut éventuellement encore contenir en plus 1 ou 2 hétéroatomes comme chaînons du noyau et qui peut éventuellement être substitué par des groupes alkyle, R₂ représente un hydrogène, un chlore ou un reste alkyle, R₃ représente un hydrogène, un reste alkyle, cycloalkyle, phényle, benzyle, pyridyle ou alcoxyalkyle et R₄ et R₅ représentent, indépendamment l'un de l'autre, un hydrogène, un reste alkyle, le reste phényle, un reste alcoxy, un halogène, un groupe amino, un groupe mono- ou dialkylamino, un groupe alkylmercapto, un hydroxy ou un reste alcoxyalkyle, de préférence ceux où R₁ représente un hydrogène, un reste (alkyle en C₁-C₄)sulfonyle, un reste phénylsulfonyle, un reste (alcoxy en C₁-C₄)sulfonyle, le reste cyano, le groupe sulfo et ses sels, le groupe carboxyle et ses sels ou un groupe de formule ―COOY'₁, ―CONY'₁Y'₂ ou ―SO₂NY'₁Y'₂, où Y'₁ et Y'₂ représentent, indépendamment l'un de l'autre, un hydrogène, un reste alkyle en C₁-C₄ ou le reste benzyle, R₂ représente un hydrogène, un chlore ou un reste alkyle en C₁-C₄, R₃ représente un hydrogène, un reste alkyle en C₁-C₄, un reste benzyle, phényle, pyridyle ou alcoxyalkyle en C₂-C₈ et R₄ et R₅ représentent, indépendamment l'un de l'autre, un hydrogène, un reste alkyle en C₁-C₄, le reste phényle, un reste alcoxy en C₁-C₄, un chlore, un reste alcoxyalkyle en C₂-C₈, un reste (alkyle en C₁-C₄)mercapto, le groupe amino, un groupe (alkyle en C₁-C₄)amino ou un groupe dialkylamino en C₂-C₆.

4. 4-hétérocyclylvinyl-4'-styryl-biphényles selon la revendication 3, où R₁ représente un hydrogène, un reste (alkyle en C₁-C₄)sulfonyle, le groupe cyano, le groupe carboxy, le reste alcoxycarbonyle en C₂-Cₛ ou aminocarbonyle, R₂ représente un hydrogène ou un chlore, R₃ représente un hydrogène, un reste alkyle en C₁-C₄, le reste benzyle, phényle, pyridyle ou alcoxyalkyle en C₂-C₆ et R₄ et R₅ représentent, indépendamment l'un de l'autre, un hydrogène, un reste alkyle en C₁-C₄, le reste phényle, un reste alcoxy en C₁-C₄, un groupe (alkyle en Ci-C₄)mercapto, le groupe amino, (alkyle en C₁-C₄)amino ou dialkylamino en C₂ -Ce.

5. 4-hétérocyclylvinyl-4'-styryl-biphényles selon une des revendications 1 à 4, où A représente un reste 1,3,4-oxadiazol-2-yle, 1,2,4-oxadiazol-5-yle, 1,2,4-oxadiazolyl-3-yle, 1,2-oxazol-3-yle, 1,2-oxazol-5-yle ou 1,3-pyrimidin-2-yle, substitué ou non substitué, de préférence un reste 1,3,4-oxadiazol-2-yle, 1,2,4-oxadiazol-5-yle ou 1,2,4-oxadiazol-3-yle.

6. 4-hétérocyclylvinyl-4'-styryl-biphényles selon la revendication 5 de formule 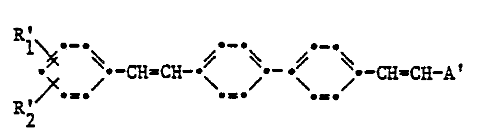 dans laquelle R'₁ représente un reste (alkyle en C₁-C₄)sulfonyle, le groupe cyano, carboxy ou un reste alcoxycarbonyle en C₂-C₅, R'₂ représente un hydrogène ou un chlore, et A' représente un reste de formule 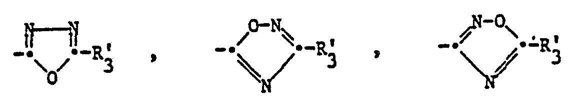 dans laquelle R'₃ représente un hydrogène, un reste alkyle en C₁-C₄ ou le reste phényle, de préférence un reste alkyle en C₁-C₄.

7. 4-hétérocyclylvinyl-4'-styryl-biphényles selon une des revendications 1 à 6, où R₁ R', représente le groupe cyano, un reste (alkyle en C₁-C₄)sulfonyle ou un reste alcoxycarbonyle en C₂-C₅, de préférence le groupe cyano, et R₂ et R'₂ représente un hydrogène.

8. Procédé de préparation des 4-hétérocyclyl-4'-styrylbiphényles ayant la formule (1) définie dans la revendication 1, caractérisé par le fait que l'on fait réagir un composé de formule 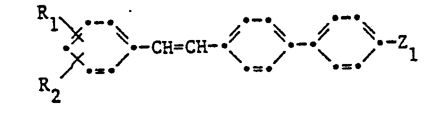 avec un composé de formule Z₂―A, ou un composé de formule 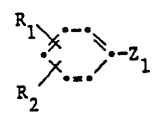 avec un composé de formule 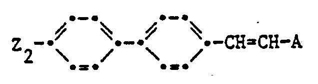 R₁, R₂ et A, dans les formules ci-dessus, étant définis comme dans la revendication 1 et à chaque fois un des deux substituants Z₁ et Z₂ représentant le groupe 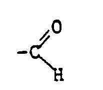 et l'autre représentant le groupe méthyle, carboxyméthyle (ou un de ses dérivés fonctionnels) ou un groupe de formule 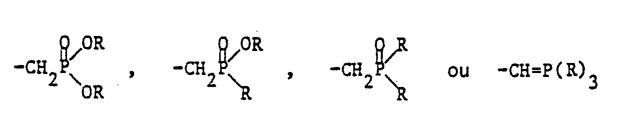 où R représente un reste alkyle en C₁-C₅, substitué ou non substitué, ou le reste phényle substitué ou non substitué.

9. Procédé de préparation des 4-hétérocyclylvinyl-4'-styryl-biphényles ayant la formule (1) définie dans la revendication 1, dans laquelle A représente un groupe de formule 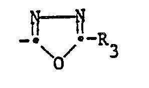 R₃ représentant un hydrogène ou un substituant non chromophore, caractérisé par le fait que l'on fait réagir un acide carboxylique de formule 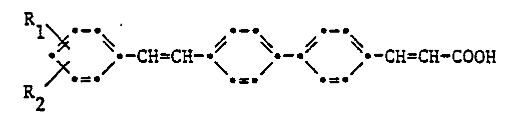 ou un dérivé fonctionnel de cet acide, avec un hydrazide d'acide carboxylique de formule H_{Z}N-NH-CO-R₃, ou un composé de formule 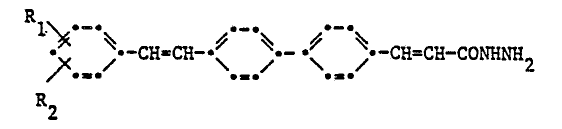 avec un acide carboxylique de formule HOOC-R₃ ou avec un dérivé fonctionnel de cet acide, R₁ et R₂ étant définis comme dans la revendication 1 et R₃ étant défini comme ci-dessus, et que l'on cyclise le produit de condensation obtenu en présence d'un agent déshydratant.

10. Procédé de préparation des 4-hétérocyclylvinyl-4'-styryl-biphényles ayant la formule (1) définie dans la revendication 1, dans laquelle A représente un groupe de formule 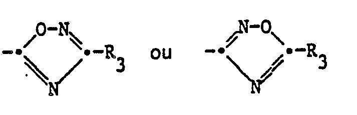 R₃ représentant un hydrogène ou un substituant non chromophore, caractérisé par le fait que l'on fait réagir un acide carboxylique de formule 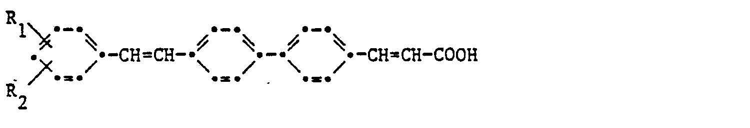 ou un dérivé fonctionnel de cet acide, avec un amidoxime de formule 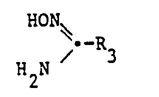 ou un composé de formule 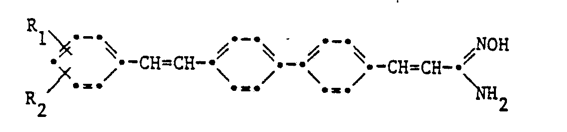 avec un acide carboxylique de formule HOOC-R₃ ou avec un dérivé fonctionnel de cet acide, R₁ et R₂ étant définis comme dans la revendication 1 et R₃ étant défini comme ci-dessus, et que l'on cyclise le produit de condensation obtenu en présence d'un agent déshydratant.

11. Agent pour l'azurage optique de matières organiques de poids moléculaire élevé, qui contient un ou plusieurs 4-hétérocyclylvinyl-4'-styryl-biphényles définis dans les revendications 1 à 7.

12. Agent selon la revendication 11, contenant ou composé d'un ou de plusieurs 4-hétérocyclylvinyl-4'-styryl-biphényles définis dans les revendications 1 à 7 et d'un ou de plusieurs azurants optiques de la classe des 1,4-bis-styrylbenzènes, 4-benzoxazolylstilbènes, 4,4'-divinylstilbènes, naphtalimides, 4,4'-bis- styrylbiphényles, 4,4'-bis-triazolyl-stilbènes, bis-benzoxazolyl-thiophènes, -naphtalènes et -éthylènes, oxadiazolyl-stilbènes, naphtotriazol-2-yl-stilbènes, triazinyl-pyrènes, 2-styrylbenzoxazoles et des coumarines, en particulier les triazolyl- ou pyrazolyl-coumarines, les azurants des classes indiquées étant de préférence des azurants de polyesters.

13. Agent selon la revendication 12, contenant, comme substance active azurante, 10 à 99 %, en particulier 30 à 70 %, d'un 4-hétérocyclylvinyl-4'-styryl-biphényle défini dans les revendications 1 à 7 et 90 à 1 %, en particulier 70 à 30 %, d'un ou de plusieurs azurants optiques des classes définies dans la revendication 12.

14. Agent selon la revendication 12, qui contient, comme azurant de polyesters, le 1,4-bis-(benzoxazol-2-yl)-naphtalène, le 1-(4'-méthoxycarbonylphényl)-2-(5',6'-diméthylbenzoxazol-2'-yl)éthylène, le 4,4'-bis-(éthoxy-carbonyl-vinyl)-stilbène, le 4,4'-bis-(cyanovinyl)-stilbène, le 1,4-bis-(2'-cyanostyryl)-benzène, le 1,5-bis-(benzoxazol-2'-yl)-thiophène, le 4-phényl-4'-(5",7"-diméthylbenzoxazol-2"-yl)-stilbène, le 1,2-bis-(5'-méthylbenzoxazol-2'-yl)-éthylène, le (4-benzoxazol-2'-yl)-4'-(3"-méthyl-1",2",4"-oxadiazol-5"-yl)-stilbène ou le 2,4-diméthoxytriazin-6-yl-pyrène, le rapport quantitatif au 4-hétérocyclylvinyl-4'-styryl- biphényle s'élevant dans chaque cas entre 1 : 2 et 2 : 1.

15. Procédé d'azurage optique de substrats organiques de poids moléculaire élevé, naturels, semi-synthétiques ou synthétiques, en particulier des fibres textiles, caractérisé par le fait que l'on incorpore aux substrats un composé défini dans les revendications 1 à 7 ou un mélange d'azurants selon une des revendications 12 à 14, ou qu'on les dépose sur les substrats, de préférence sur des fibres textiles, les substrats à azurer se composant en particulier de polyester ou de polyamide.

16. Procédé selon la revendication 15, caractérisé par le fait que l'on incorpore au substrat à azurer ou que l'on dépose sur ce substrat de 0,001 à 2 %, de préférence de 0,01 à 0,5 % de l'azurant ou du mélange d'azurants, par rapport au poids de substrat.

17. Utilisation des composés définis dans les revendications 1 à 7 pour l'azurage optique des matières organiques de poids moléculaire élevé, naturelles, semi-synthétiques ou synthétiques, en particulier en polyester ou en polyamide, avant tout en fibres de polyester.
